(19) Europäisches Patentamt
European Patent Office
Office européen des brevets

(11) EP 3 724 209 B1

(12) **EUROPEAN PATENT SPECIFICATION**

(45) Date of publication and mention of the grant of the patent:
**15.09.2021 Bulletin 2021/37**

(21) Application number: **18833326.4**

(22) Date of filing: **14.12.2018**

(51) Int Cl.:
***C07K 7/06*** *(2006.01)* ***A61L 27/22*** *(2006.01)*
***C07K 14/78*** *(2006.01)* ***C07K 14/435*** *(2006.01)*
***C08L 89/00*** *(2006.01)*

(86) International application number:
**PCT/IB2018/060109**

(87) International publication number:
**WO 2019/116342 (20.06.2019 Gazette 2019/25)**

(54) **ELASTOMERIC PEPTIDE**

ELASTOMERES PEPTID

PEPTIDE ÉLASTOMÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2017 IT 201700144956**

(43) Date of publication of application:
**21.10.2020 Bulletin 2020/43**

(73) Proprietor: **Politecnico di Milano**
**20133 Milano (IT)**

(72) Inventors:
- **METRANGOLO, Pierangelo**
  **20133 Milano (IT)**
- **BALDELLI BOMBELLI, Francesca**
  **20133 Milano (IT)**
- **PIZZI, Andrea**
  **20133 Milano (IT)**
- **LINDER, Markus**
  **00076 Aalto (FI)**
- **MOHAMMADI, Pezhman**
  **02150 VTT (FI)**

(74) Representative: **Croce, Valeria et al**
**Jacobacci & Partners S.p.A.**
**Via Senato, 8**
**20121 Milano (IT)**

(56) References cited:
**WO-A1-2004/104043 WO-A2-2009/069123**

- **H. EDWARD WONG ET AL: "Halogenation Generates Effective Modulators of Amyloid-Beta Aggregation and Neurotoxicity", PLOS ONE, vol. 8, no. 2, 28 February 2013 (2013-02-28), page e57288, XP055479891, DOI: 10.1371/journal.pone.0057288**
- **SHENG-CHEN HUANG ET AL: "Rational Design and Hierarchical Assembly of a Genetically Engineered Resilin-Silk Copolymer Results in Stiff Hydrogels", ACS BIOMATERIALS SCIENCE & ENGINEERING, vol. 3, no. 8, 22 June 2017 (2017-06-22), pages 1576-1585, XP055479788, US ISSN: 2373-9878, DOI: 10.1021/acsbiomaterials.7b00353**
- **ANGELO BRACALELLO ET AL: "Design and Production of a Chimeric Resilin-, Elastin-, and Collagen-Like Engineered Polypeptide", BIOMACROMOLECULES, vol. 12, no. 8, 8 August 2011 (2011-08-08) , pages 2957-2965, XP055236188, US ISSN: 1525-7797, DOI: 10.1021/bm2005388**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 724 209 B1

## Description

The invention concerns an oligopeptide based on a consensus sequence that is recurrent in natural bioelastomers, which has been chemically modified to display efficient elastomeric behavior.

### Background

Bioelastomers are usually polymeric materials mainly used to fabricate scaffolds for tissue engineering and drug delivery. They are also used in cosmetic formulations, as well as for advanced polymer manufacturing, such as micromachining. Bioelastomers possess three distinctive features:

1) three-dimensional network, similar to that of natural elastin, formed through cross-linking among polymer chains;
2) high flexibility and elasticity, matching the mechanical properties of soft body tissues;
3) biocompatibility and biodegradability.

Usually, bioelastomers are macromolecules, i.e., high polymers and proteins. In general, chemical cross-linking improves the mechanical properties of these materials inducing thermoplastic behavior. Chemical cross-linking, in fact, confers high elasticity and shape stability to bioelastomers by reducing the relaxation and creep of molecular chains. Although the significant mechanical improvements provided by chemical-crosslinking, biocompatibility and bioactivity are usually reduced by this process and manufacturing costs are instead increased. Moreover, the high molecular weight of these materials increases costs of synthesis and purification.
Different classes of bioelastomers are available as well as methods to produce them. Regarding recombinant DNA technologies, cloning a gene into a bacterial vector to obtain recombinant proteins implies remarkable efforts, both in term of time and costs, especially for the purification step. This is also the case for chemical synthesis, especially when the protein sequence is very long.
Resilin, an insect natural protein showing rubber-like elasticity, and, more in general, natural occurring proteins showing elastomeric properties, are used in combination with structural proteins such as silk fibroin, keratin, and collagen for obtaining hybrid materials with enhanced mechanical and elastic properties. In the last 15 years, Resilin-like polypeptides (RLPs) have been developed.
The first example of RLP was obtained by expressing the *Drosophila melanogaster* gene CG15920 (Elvin CM et al., Synthesis and properties of crosslinked recombinant pro-resilin. Nature 2005, 437, 999-1002). The obtained protein, named Rec1-resilin, includes 17 repetitions of the sequence SEQ ID no. 1 GGRPSDSYGAPGGGN, said sequence SEQ ID no. 1 being the elastic motif of resilin. The material becomes rubber-like upon photo-cross-linking mediated by Ru(II), and revealed to be highly pH and temperature responsive. This stimuli-responsive behavior determined the development of Rec1-resilin as a component in biosensors and diagnostic tools (Truong MY et al., A pH-responsive interface derived from resilin-mimetic protein Rec1-resilin. Biomaterials 2010, 31, 4434-4446).
As a further example, RLPS comprising 12 repeats of the resilin consensus sequence, referred to as RLP12, has been applied to bio rubber, wherein RLP12 yields materials with elastomeric properties upon chemical cross-linking involving the lysine residues and the cross-linker β-[tris(hydroxymethyl)phosphino]-propionic acid, THPP (Charati MB et al., Hydrophilic elastomeric biomaterials based on resilin-like polypeptides. Soft Matter 2009, 5, 3412-3416).
Other repetitive consensus sequences derived from different insects were used to generate alternative resilin-like materials. The protein An16, obtained from *Anopheles gambiae* resilin, contains 16 repetitions of the fragment SEQ ID no. 2 AQTPSSQYGAP, while the protein Dros16, obtained from *D. melanogaster* resilin, contains 16 repetitions of the sequence SEQ ID no. 3 GGRPSDSYGAPGGGN (Tamburro AM et al., Localizing α-Helices in Human Tropoelastin: Assembly of the Elastin "Puzzle". Biomacromolecules 2009, 10, 3009-3014). Both An16 and Dros16 showed, upon photo-cross-linking, resilience and Young's moduli comparable to those of Rec1-resilin.
The resilin motifs of An16 were subsequently included into a modular protein having a cell-binding domain (RGD) for tissue engineering applications related to cartilage regeneration (Renner JN et al., Characterization of Resilin-Based Materials for Tissue Engineering Applications. Biomacromolecules 2012, 13, 3678-3685). The growing interest in multifunctional material, where the structural properties of different proteins are condensed into a unique component, led to the production of hybrid polypeptides. Research in this field focused on chimeric resilin-elastin-collagen-like polypeptides (REC). These hybrid proteins contain a resilin consensus sequence, SEQ ID no. 4 SDTYGAPGGGNGGRP, an elastin fragment and a collagen domain (Flamia R et al., AFM Study of the Elastin-like Biopolymer Poly(ValGlyGlyValGly). Biomacromolecules 2011, 12, 2957-2965). Said polypeptides show an elastomeric behavior near to ideality. Moreover, mesenchymal stem cells (MSCs) were found to adhere and proliferate on surfaces coated with RECs, suggesting the potential use of these chimeric proteins in driving cell-growth and cell differentiation (Sbrana F et al., Multiscale characterization of a chimeric biomimetic polypeptide for stem cell culture. Bioinspir. Biomim. 2012, 7, 046007).
WO2004/104042 discloses a recombinant polypeptide expressed from exon-1 of resilin gene of *D. melanogaster*

forming a bioelastomer upon enzymatic or photo-induced cross-linking. The amino acid sequence is more than 300 residues long.

US2010/0015070 discloses the use of natural, recombinant and synthetic resilin proteins in skin cosmetics. The materials used for dermatological compositions contains up to 100 repeat units, wherein said repeat unit is SXXYGXP (SEQ ID no. 5), where X is a charged or polar amino acid. The resilin motifs are part of a hybrid protein, where structural domains derived from silk fibroin, keratin elastin or collagen are combined in the same sequence.

US2016/0279295 deals with the use of a resilin polypeptide, in combination with other polymeric moieties (collagen, PEG, etc.), to provide an article of manufacturing for treating tissue damage or loss. The resulting polypeptide, after cross-linking, is in a form of plurality of spherical particles (diameter range 1-200 μm) or in a form of a membrane.

WO 2004/104043 discloses a bioelastomer comprising a plurality of repeats units with the consensus motif SXXYGXP, wherein Y is any amino acid.

WO 2009/069123 discloses a resilin polypeptide comprising at least two repeating units SXXYGYXP, wherein X is represented by any amino acid.

From the above, it appears clearly that state of the art polypeptides, both natural and synthetic, are either very long or combined with synthetic polymeric strands to exert the desired elastomeric behavior.

There is a strong need for an oligopeptide having elastomeric behavior, wherein short chain peptides do have an advantage over long chain peptides in that the smaller chain size is more stable. Small chain peptides do not experience some of the issues that long chain peptides do because of their shorter length, most notably collapsing during absorption. Additionally, short chain peptides can be replicated much more efficiently (Shin H et al., Biomimetic materials for tissue engineering. Biomaterials, 2003 24:4353-5364).

## Summary of the invention

The scope of the invention is defined in the appended set of claims.

## Detailed description

Oligopeptide derivatives based on a consensus sequence that is recurrent in natural bioelastomers have been designed.

Through conventional peptide synthesis, a series of modified wild-type resilin consensus sequences have been produced, wherein said oligopeptides have been modified through selective chemical halogenation of specific amino acid residues. The obtained short halogenated peptides, surprisingly, showed interesting elastic structural properties not encoded in the unmodified peptides.

## Figures description

Figure 1: Physical aspect of 100 mM solutions of oligopeptide SDS-Y(3,5Br)-GAP (A) and SDYGAP (B, comparative).

Figure 2: (A) Rod-like crystals obtained from a 100 mM solution of peptide SDSYGAP (SEQ ID no. 6), comparative, and (B) Fibril network imaged from a 100 mM solution of an oligopeptide according to the present invention, i.e. SDS-Y(3,5 Br)-GAP.

Figure 3: Resilient, macroscopic fibers stretched from a 100 mM solution of an oligopeptide according to the present invention, i.e. SDS-Y(3,5 Br)-GAP.

Figure 4: Congo red staining of a 100 mM solution of an oligopeptide according to the present invention, i.e. SDS-Y(3,5 Br)-GAP.

Figure 5: (A, B) TEM images of a 10 mM solution of peptide SDSYGAP (SEQ ID no. 6), comparative, (C, D) TEM images of a 10 mM solution of an oligopeptide according to the present invention, i.e. SDS-Y(3,5 Br)-GAP, (E-H) TEM images of SDS-Y(3,5 Br)-GAP 100 mM after 2 days aging, (I, L) SEM images of SDS-Y(3,5 Br)-GAP 100 mM after 2 days aging.

Figure 6: (A) FTIR spectra of a dried solution of peptide SDSYGAP (10 mM), comparative, (B) FTIR spectra of a dried solution of SDS-Y(3,5 Br)-GAP (10 mM).

Figure 7: (A) Autocorrelation functions of SDSYGAP (10 mM) in water at 90°, comparative, (B) Autocorrelation functions of SDS-Y(3,5 Br)-GAP (10 mM) in water at 90°.

Figure 8: (A) CD spectra of peptide SDSYGAP (5 mM) in water at different aging times, comparative, (B) CD spectra of SDS-Y(3,5 Br)-GAP (5 mM) in water at different aging times.

Figure 9: (A) 1D SAXS/WAXS profile of resilin peptides, (B) Diffraction pattern of SDS-Y(3,5 Br)-GAP (100 mM).

Figure 10: XRPD spectra (A) background: silicon crystal, (B) SDSYGAP comparative, (C) SDSY(3,5-Br)GAP.

Figure 11: Cell viability assay after exposure to (A) SDSYGAP, comparative, (B) SDSY(3,5-Br)GAP.
Figure 12: elastin gene expression profile in hSF exposed to SDS-Y(3,5 Br)-GAP
Figure 13: A) Cell proliferation and B) protein synthesis in hSF exposed to SDS-Y(3,5 Br)-GAP
Figure 14: A) ROS inhibition and B) cell viability in HuKe exposed to SDS-Y(3,5 Br)-GAP
Resilin exon I comprises a consensus sequence, SEQ ID no. 6 SDSYGAP, represented by Formula (I)

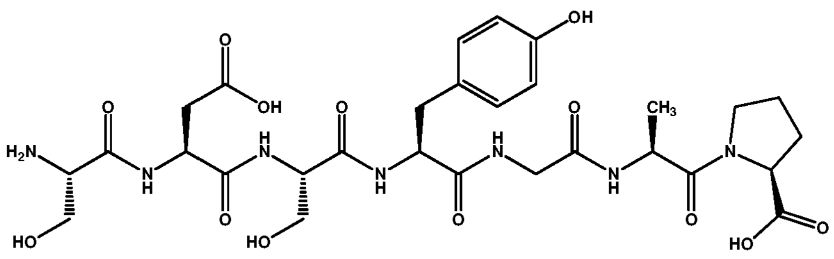
(I)

Here it is described the obtainment of oligopeptides wherein the elastomeric properties known for polymeric systems containing the indicated consensus sequence SEQ ID no. 6 are surprisingly encoded in a non-polymeric system, i.e. within the single oligopeptide molecule.

The surprising results were obtained through a chemical modification of an oligopeptide, i.e., by substituting with at least one halogen atom at least one residue on said oligopeptide, wherein said residue to be substituted is selected from the group consisting of: tyrosine, histidine, phenylalanine, tryptophan.

The oligopeptide according to the present disclosure is SJXXZGXP (SEQ ID no. 7), where S is a serine, J is a serine or it is absent, X is independently any desired amino acid, Z is a tyrosine, histidine, phenylalanine or tryptophan, G is a glycine and P is a proline, wherein at least the residue Z is modified with at least one halogen selected from Br, Cl, I.

In an embodiment here disclosed, in said oligopeptide SJXXZGXP (SEQ ID no. 7) S is a serine, J is absent, X is independently any desired amino acid, Z is a tyrosine, histidine, phenylalanine or tryptophan, G is a glycine and P is a proline, wherein at least the residue Z is modified with at least a halogen atom selected from Br, Cl I.

In a further embodiment here disclosed, in said oligopeptide SJXXZGXP (SEQ ID no. 7) S is a serine, J is absent, X is independently any desired amino acid, Z is a tyrosine, G is a glycine and P is a proline, wherein at least the residue Z is modified with at least a halogen atom selected from Br, Cl I.

In a further embodiment here disclosed, in said oligopeptide SJXXZGXP (SEQ ID no. 7) S is a serine, J is a serine or it is absent, X is independently any desired amino acid, Z is a tyrosine, G is a glycine and P is a proline, wherein the phenol on said tyrosine Z is mono or di-ortho-halogenated, preferably it is di-ortho-brominated.

In a further embodiment here disclosed, in said oligopeptide SJXXZGXP (SEQ ID no. 7) S is a serine, J is absent, X is independently any desired amino acid, Z is a histidine, G is a glycine and P is a proline, wherein the imidazole on said histidine Z is mono or di-halogenated, preferably it is di-brominated.

In a further embodiment here disclosed, in said oligopeptide SJXXZGXP (SEQ ID no. 7) S is a serine, J is absent, X is independently any desired amino acid, Z is a phenylalanine, G is a glycine and P is a proline, wherein the phenyl on said phenylalanine Z is mono para-halogenated, preferably it is iodinated.

In a further embodiment here disclosed, in said oligopeptide SJXXZGXP (SEQ ID no. 7) S is a serine, J is absent, X is independently any desired amino acid, Z is a tryptophan, G is a glycine and P is a proline, wherein the indole on said tryptophan Z is mono or di-halogenated, preferably it is di-brominated. According to claim 1, said oligopeptide is SDSY*GAP (SEQ ID no. 8) wherein S is a serine, D is aspartic acid, Y is tyrosine, G is a glycine, A is alanine and P is a proline, wherein * is indicative of the fact that said tyrosine is mono or di-halogenated, preferably it is di-brominated, still more preferably it is di-ortho-brominated, i.e., it is SDS-Y(3,5 Br)-GAP.

It has surprisingly been observed that selective halogenation deeply affects the properties of the oligopeptide, wherein the elastomeric behavior observed in the halogenated oligopeptides according to the present description were not observed in the wild-type sequences. Halogen atoms are well-known substituents able to promote peptide self-assembly in amyloidogenic peptides and proteins (Bertolani A et al., Supramolecular amplification of amyloid self-assembly by iodination. Nature Communications 2015, 6:7574), however the use of halogenation as a strategy to induce elasticity in peptide-based bioelastomers has never been disclosed.

Here it is demonstrated that the presence of halogen atoms in the oligopeptide of the invention has the following main effects:

1. it decreases the peptide solubility, thus facilitating the formation of elastomeric structures;
2. it amplifies the non-covalent interaction pattern involved in peptide self-assembly, which promotes the formation of complex and less soluble superstructures, i.e., a physical cross-linking;

3. it increases peptide chemical stability;
4. it introduces the possibility of further chemical modifications at the halogenated functional groups.

In the experimental section here below reported, data obtained from exemplificative halogenated oligopeptides according to the claimed invention are reported, compared to the observation made when using the naturally occurring consensus sequence SEQ ID no. 6. Data are intended to be exemplificative and not limitative for the claimed invention.
The halogenated oligopeptides self-assemble in water into macroscopic birefringent fibers, as observed by Polarized Light Optical Microscopy. Green birefringence of these fibers was observed upon staining with Congo Red, suggesting a certain degree of amyloid structure, therefore confirming that the here claimed halogenated oligopeptides intrinsically possesses both rigid and elastomeric properties, unusually matched in a single peptide sequence.
Small-angle X-ray diffraction analysis confirmed the ordered supramolecular organization of these fibers, not observed in the naturally occurring oligopeptide. Moreover, macroscopic elastic fibers can be stretched from aged aqueous solution of the halogenated oligopeptides.
Since biocompatibility is necessary for a product suitable for biomedical applications, cytotoxicity assays were performed, confirming that the presence of halogen atoms does not affect the toxicity of the claimed oligopeptides, even at high concentrations.
The advantage of using halogenated oligopeptides with respect to conventional elastomeric protein and polymer materials lies in their easy synthetic and purification processes, wherein said halogenated oligopeptides are obtained by standard peptide synthesis, therefore making their production easily scalable at a reduced cost.
The elastomeric properties of the here described halogenated oligopeptides emerge by spontaneous self-assembly, without the need of chemical cross-linking.
In a further embodiment, it is here claimed the use of the halogenated oligopeptides according to claim 1 or 2 in biomedical applications. As an example, said halogenated oligopeptides are used advantageously as scaffolds in tissue engineering, cosmetic, regenerative medicine, e.g., artificial vessels, vascular prostheses, wound healing, and joint reconstruction, repair, and cushioning.
Fibroblasts are the main cell component in the connective tissue of the dermis and they are able to synthesize high amount of collagen and elastin. The ability of the tested halogenated oligopeptides to promote collagen I and elastin neo-synthesis in fibroblasts, is indicative of their role in supporting the skin tropism and the dermis fibres synthesis and/or in counter-acting the dermis loss of tone and structure, typically observed in skin ageing and following environmental or hormonal stresses.
Moreover, the halogenated oligopeptides have been demonstrated to play a role on the stimulation of the cell cycle and on the total protein synthesis in human fibroblasts. In a further embodiment, it is here claimed the use of the halogenated oligopeptides according to claim 1 or 2 as high-efficiency industrial elastomers, thermoplastics, biocomposite, and nanoparticle matrices.
A further use is as components in cosmetic formulations, where the halogenated oligopeptides according to claim 1 or 2 act as filler, film-forming and/or moisturizer.
In a further embodiment, the halogenated oligopeptide and the fibers from the same are applied in the production of nanosprings, nanomachines and biosensors.

## EXAMPLES

**Example 1:** solubility test

The isoelectric point (calculated on http://web.expasy.org/compute-pi/) of SDSYGAP (SEQ ID no. 6), comparative, and an oligopeptide according to the present invention, i.e., SDS-Y(3,5 Br)-GAP is 3.8. Three buffers at pH 3, 7, and 12 (10 mM concentration) and pure water were used as solvent for the two oligopeptides. The two oligopeptides showed good solubility properties in all the tested conditions, wherein 100 mM solutions (5 mg in 50 $\mu$l) are completely clear after heating. However, the brominated oligopeptide solution becomes milky after cooling, while the wild type sequence solution is still clear after cooling, as showed in Figure 1, panel A and B, respectively. This indicates that the presence of the halogen atoms decreases the solubility of the oligopeptide.

**Example 2:** formation of an entangled fibril network

Dried 100 mM solutions of wild type and halogenated peptide according to Example 1 were observed with a polarized optical microscope and images were registered with a Leica DM4500P POM system, equipped with a Canon EOS 60D camera. Figure 2A shows the images observed for the wild type oligopeptide, Figure 2B for the halogenated one. An entangled fibril network is clearly observed only for the brominated oligopeptide. The wild type oligopeptide forms, upon drying, rod-like crystals trapped in a film of amorphous material.

Moreover, it was observed that only fibers formed by the halogenated oligopeptide were able to rehydrate by adding fresh solvent. The rehydrated fibers regained elasticity so that it was possible to stretch some of them by using a needle. Resilient, macroscopic fibers stretched from the rehydrated solution of the halogenated peptide are shown in Figure 3.

**Example 3:** fibrils contain amyloid domains

The samples were monitored for green birefringence using a Leica DM4500P POM system equipped with a Canon EOS 60D camera. A drop of each oligopeptide solution, i.e. wild type and halogenated (100 mM) was placed on a glass microscope, allowed to air dry and then stained with Congo Red, wherein a 80% ethanol solution saturated with NaCl and Congo Red was freshly prepared before each measurement. Subsequently, excess Congo Red solution was blotted off the slide and the samples were analyzed using both bright and polarized light. An exemplificative image for the halogenated oligopeptide is reported in Figure 4.

Congo red staining indicates the presence of amyloid domains in the fibril formed by the halogenated oligopeptide. No green birefringence is observed when staining the solution of the wild type oligopeptide (not shown).

**Example 4:** halogenated oligopeptide forms complex self-assembled structures

Transmission Electron Microscopy (TEM) bright field images were acquired using a Philips CM200 electron microscope operating at 200 kV equipped with a Field Emission Gun filament. A Gatan US 1000 CCD camera was used and 2048x2048 pixels images with 256 grey levels were recorded. The samples were dropped onto a 200 mesh carbon-coated copper grid and air dried for several hours before analysis. Exemplificative images are reported in Figure 5. Halogenated oligopeptide, as shown in panels C, D, forms self-assembled structures with higher complexity and hierarchy with respect to those observed with the wild type, panels A, B. Moreover, the aggregation process is faster when the oligopeptide is halogenated: after 2 days wild type oligopeptide forms amorphous aggregates (panel A), while the halogenated oligopeptide forms distinct spherical structures (panel C). Morphology of the self-assembled structures is not strictly dependent on sample concentration. Repeating the experiment using 100 mM solutions (TEM: panels E-H, SEM: panels I, L) the results overlap the results observed operating at 10 mM.

**Example 5:** halogenation confers both strength (β-sheet) and elasticity (β-turn)

Infrared spectra were recorded at room temperature using a Nicolet iS50 FT-IR spectrometer equipped with a DTGS detector.

Oligopeptides were analyzed as dried solutions or bulk powders. Spectra reported in Figure 6A and 6B represent an average of 64 scans recorded in a single beam mode with a 4 $cm^{-1}$ resolution and corrected for the background. The second derivative analyses of the spectra were performed using the Nicolet FTIR software, Omnic 9.0®, with a 13-point and $3^{rd}$ polynomial order Savitzky and Golay function. Second derivative spectra generated negative bands as compared with the original spectra, thus for comparison all the second-derivative spectra (red line in the figures below) were multiplied by -1.

The amide I region of the FTIR spectra (1600-1700 $cm^{-1}$) gives evidence of the predominant structural motif characterizing the self-assembly of wild type (6A) and halogenated (6B) oligopeptides. Halogenated oligopeptide, besides the peak related to β-sheet (peculiar structural motif of amyloids) shows a signal at 1667 $cm^{-1}$ that can be associated to β-turn, a typical secondary structure formed by elastomeric peptides. FTIR of halogenated oligopeptide confirms that the presence of bromine atoms confers both strength (β-sheet) and elasticity (β-turn).

**Example 6:** decay time as a dimensional indicator of the formed structures

Dynamic Light Scattering measurements were performed on an ALV/CGS-3 Platform-based Goniometer System equipped with an ALV-7004 correlator and an ALV/CGS-3 goniometer. The signal was detected by an ALV-Static and Dynamic Enhancer detection unit. The light source was the second harmonic of a diode-pumped Coherent Innova Nd:YAG laser (λ = 532 nm), linearly polarized in the vertical direction. Measurements were performed at 25°C. Approximately 1 ml of sample solution was transferred into the cylindrical Hellma scattering cell.

The dynamic information on structures present in the peptide solutions were derived from the normalized autocorrelation function $g_2$ (q, τ) of the scattered intensity, which is measured according to

$$g_2(q,\tau) = \frac{\langle I(q,t) I(q,t+\tau) \rangle}{\langle I(q,t) \rangle^2}$$

where q is the scattering vector, $\tau$ is the relaxation time and I is the scattered intensity. Higher relaxation times correspond to larger self-assembled structures. Data analysis has been performed through Laplace inversion (CONTIN algorithm), which resulted in a double distribution of the decay rates associated to two distinct populations. Results are reported in Figure 7, as graphs showing the autocorrelation functions of wild type (panel A) or halogenated (panel B) oligopeptide 10 mM in water at 90°C. The autocorrelation functions at 90°C show increasing Lag Time, i.e., increasing dimensions of the self-assembled structures, over time. Table 1 summarizes and compares the results, showing that halogenated oligopeptide form bigger structures compared to the wild type one. This is a further indication that bromine atoms play a role in the aggregation behavior of the peptide sequence. The same aging study was performed in buffer at pH 7, with similar results (data not shown).

Table 1

| Aging time | Lag time wild type (ms) | Lag time halogenated (ms) |
|---|---|---|
| t=0 | 1.512 | 2.647 |
| t=24 h | 2.859 | 6.751 |
| t=48 h | 1.919 | 8.283 |
| t=1 week | 3.826 | 11.08 |

**Example 7:** circular dichroism

Circular dichroism (CD) experiments were carried out in a 0.05 mm quartz cuvette, using a JASCO J-815 CD spectrometer. Acquisitions were performed between 190 and 250 nm with a 0.1 nm data pitch, 1 nm bandwidth, 100 nm $min^{-1}$ scanning speed and 1 s response time. Spectra reported in Figure 8A for the wild type and 8B for the halogenated oligopeptide are an average of 10 scans and were corrected from a reference solution, consisting of deionized water (18.2 M$\Omega$.cm). Raw data ($\theta$, in mdeg) were subsequently converted to mean residue ellipticity ([$\theta$] in deg.$cm^2$.$dmol^{-1}$) for the sake of comparison, in accordance with the following formulae:

$$[\theta] = \frac{\theta}{10 * l * c * (n-1)}$$

where $\theta$ is the observed ellipticity in mdeg, c is the concentration of the sample in mol.$l^{-1}$, (n-1) is the number of peptide bonds, and 1 is the pathlength of the cuvette in cm.

Both CD signature of wild type and halogenated oligopeptide can be related to a polyprolineII (PPII) secondary structure, which is peculiar in elastomeric compounds. The negative peak in the region 190-200 nm is red-shifted in the CD spectra of the halogenated compound, indicating that the presence of bromine stabilizes the charge-transfer transition ($\pi$-$\pi$*) related to the peptide bond. This is probably due to the high polarizability of bromine, which behaves as electrophilic species because of the anisotropic distribution of the electron density on its surface. The CD signature of both oligopeptides do not evolve overtime; however, after 10 days of aging there is a drop of the signal related to the halogenated peptide, suggesting a partial aggregation and precipitation of the peptide. This finding further confirms that bromine enhances insolubility, hence the aggregation propensity of the peptide. The same aging study was performed in buffer at pH 7, with similar results (data not shown).

**Example 8:** Small and Wide Angle X-Ray Scattering (Saxs/Waxs)

Wide-angle X-ray diffraction (WAXS) was performed at pSpot beamline at BESSY II synchrotron source (Berliner Elektronenspeicherring-Gesellschaft für Synchrotronstrahlung, Helmholtz-Zentrum Berlin, Germany). For all the sample was used a multilayer monochromator, with X-ray wavelength for Si (111) of 0.82656Å (15 keV), beam size of 20-50 $\mu$m, and sample-to-detector distance of 310 mm. Beamline calibration was carried out with a quartz ($SiO_4$) standard. Two-dimensional CCD detector (MarMosaic 225, Mar USA, Evanston USA) with pixel size of 73.242 $\mu$m and resolution of 3072 $\times$ 3072 pixels was used to collect the data.

A 100 mM solution of the halogenated oligopeptide showed clear diffraction, as expected. All the other samples (100 mM solution of wild type oligopeptide and samples at 10 mM) and pure water show exactly the same diffraction except for a slight difference in diffraction intensity.

Diffraction associated to the 100 mM solution of halogenated oligopeptide can be divided in two distinct regions, as reported in Figure 9.

In region 1 there is a detectable diffraction at 22.74 Å, corresponding to the cell length of the halogenated peptide.

In region 2 there are multiple diffraction peaks in the range 4.19-4.67 Å. This usually arises from spacing between hydrogen-bonded β-strands within β-sheets. These results confirm that halogenated oligopeptide shows high propensity to form amyloid domains.

**Example 9:** X-Ray Powder Diffraction (XRPD) measurements

X-Ray Powder Diffraction (XRPD) measurements were performed with a Bruker AXS D8 powder diffractometer, using the following experimental parameters: Cu-Kα radiation ($\lambda$ = 0.15418 nm); scanning interval: 5 - 40° 2θ; step size: 0.015°; exposure time: 1.3 s per step. The structural features of the lattice were calculated by using the Bragg's law: $n\lambda = 2d\sin\theta$.

Results are reported in Figure 10 (A, control; B, wild type oligopeptide; C, halogenated oligopeptide). The distances calculated from the spectrum of wild type oligopeptide cannot be associated to any particular structural motif (panel B). Conversely, distances calculated from the spectrum of the halogenated oligopeptide are typical of the cross β structure in amyloid domains. The signal around 10 Å corresponds to the distance between facing β-sheets. The peaks around 4 Å can be associated to the inter-strand distance in the same β-sheet

This result is a confirmation of the previous SAXS/WAXS experiments: bromination amplifies the peptide propensity to self-assemble into amyloid domains.

**Example 10:** halogenation does not impact biocompatibility

HeLa (Human cervical cancer cells) were grown in DMEM supplemented with 1 mM sodium pyruvate, 10 mM HEPES buffer, 100 U/mL penicillin, 0.1 mg/mL streptomycin, 2 mM glutamine and 10% FBS (hereafter referred to as complete medium), at 37°C in a 5% $CO_2$ humidified atmosphere. Cells were splitted the day before seeding and used within eight passages. The cells were seeded at a density of 7000 cell/$cm^2$. For evaluating cell viability, the day after the seeding the cell medium was replaced with medium containing the wild type or the halogenated oligopeptides and cells were incubated for 24 hours. Control cells were treated with comparable amounts of PBS and cell viability analysis was carried out by PROMEGA CellTiter-Glo® Luminescent Cell Viability Assay. Results are reported in Figure 11. The cell viability assay confirms that both oligopeptides are not toxic even at high concentrations. Bromination does not affect biocompatibility.

**Example 11:** halogenation does not impact ecocompatibility

An ecotoxicity test on *Pseudokirchneriella sub capitata* was performed to evaluate I% (inhibition percentage effect) and IC50 (concentration which determines the 50% inhibition of growth on the population) of the halogenated peptide SDS-Y(3,5 Br)-GAP.

The halogenated peptide SDS-Y(3,5 Br)-GAP was dissolved in water at 100 mg/l and tested at the following concentrations: 50 mg/l, 25 mg/l, 12.5 mg/l and 6.25 mg/l. Each sample was tested in triplicate.

Potassium dichromate (IC50 = 0.31 mg/1) has been used as a positive control.

After 72 hours of incubation with the sample or controls, a spectrophotometric test was performed at 670nm as a measure of cell density.

The results are reported in Table 2 below.

Table 2

| | **Durata del test / Assay time 72h** | | | | |
|---|---|---|---|---|---|
| | **500mg/l** | **250mg/l** | **125mg/l** | **62.5mg/l** | **31.25mg/l** |
| **Media I% Medium I%** | **100%** | **100%** | **55%** | **54%** | **48%** |

The observed IC50 is 54 mg/l.

The obtained data demonstrates the tested peptide is not ecotoxic.

**Example 12:** elastin gene expression profile evaluation on human fibroblasts

Elastin gene expression in human skin fibroblasts (hSF) has been evaluated by Real Time Polymerase Chain Reaction (qRT-PCR) with and without treatment with the peptide SDS-Y(3,5 Br)-GAP.

Cells were seeded in 24 wells plates in MEM containing 10% FBS and antibiotics and incubated 24 hours.

Fresh medium was then added, supplemented with the tested peptide, dissolved directly in the culture medium.

Untreated cells were used as negative control and cells exposed to 100pg/ml of human insulin were used as positive control. Every sample was tested in three replicates. After 24 and 48 hours of exposure, total RNA was extracted. After precipitation and centrifugation (30' 12000rpm, 4°C), RNA was suspended in 20μl of sterile water and its concentration determined with a spectrophotometer.

250 ng of total RNA were retro-transcribed into cDNA using random primers at 42°C for 30h, following manufacture's instruction (Applied Biosystems, Foster City, CA). Changes in gene expression profile were analyzed with RealTime PCR technique, using SYBR Green assay.

Changing in gene expression profile were measured using the comparative $C_T$ method ($\Delta\Delta C_T$ method).

Sample data were normalized to level of expression of actin as housekeeping gene.

The difference between the $C_T$ value of the target (elastin) and the $C_T$ of the housekeeping gene (actin) was assessed.

$$\Delta C_T = C_T\ (\text{target}) - C_T\ (\text{housekeeping})$$

$\Delta C_T$ was calculated for each sample.

Untreated sample was considered as the reference (calibrator), and used for each comparation.

ΔΔCT was assessed as the difference between the $\Delta C_T$ of each sample and the $\Delta C_T$ of the untreated sample (calibrator).

$$\Delta\Delta C_T = \Delta C_T\ \text{sample 1} - \Delta C_T\ \text{untreated sample (calibrator)}$$

$\Delta\Delta C_T$ was then used to calculate Fold Change values:

$$\text{Fold change} = 2^{-\Delta\Delta CT}$$

The obtained results are shown in Figure 12. Fold change value observed by treatment with the halogenated oligopeptide at 500 pg/ml is statistically significant, therefore demonstrating the capability of the same to increase the expression of messenger RNA codifying for elastin in human fibroblasts, when compared to untreated cell.

**Example 13:** mitogenic activity evaluation

hSF were seeded in 24 wells plates and allowed to grow in MEM containing 10% FBS and antibiotics at 37°C and 5% $CO_2$ for 24 h. Cells underwent starvation in serum-free medium for 6 hours before treatment. Fresh medium without serum and supplemented with serial dilutions of the halogenated oligopeptide SDS-Y(3,5 Br)-GAP was then added to the cells. The oligopeptide was dissolved directly in the medium culture. The test was carried out in three replicates. After 24 and 48 hours exposure, cell viability and total cellular protein content were evaluated by MTT and Bradford assays on separate plates. Untreated cells were used as negative controls, cells treated with human insulin were used as positive control.

The results are shown in Figures 13 A, B and demonstrate the halogenated oligopeptide is able to stimulate protein synthesis, the highest effect being observed at 0,5 mg/ml, after 48h exposure.

**Example 14:** in vitro evaluation of protective effects against UVA-induces oxidative stress in cell cultures

The test is carried out on human keratinocytes (HuKe). Cells were seeded in 96-well plates up to semi-confluence. Once 60-70% confluent, fresh medium is added with scalar dilutions of the tested halogenated oligopeptide SDS-Y(3,5 Br)-GAP, ranging from 300 to 9 pg/ml. The oligopeptides is dissolved directly in the culture medium. Untreated cells are used as negative control. Cells treated with vitamin C at 150 pg/ml act as positive control. For each dilution, 3 replicate tests were performed. A fraction of the cells was checked for their vitality with the NRU assay. The remaining cells were then exposed to UVA irradiation. At the end of the exposure period, the ROS formation is investigated through DCFH-DA (2-7-Dichlorodihydrofluorescein diacetate).

The cell culture medium is removed and the cells are washed in PBS. The Dichlorofluorescein acetate (DCA) solution is added to each well. DCA, reacting with free radicals in the medium, originates a fluorescent derivative, and the fluorimeter reading allows obtaining a quantitative data related to the ROS content in the cells. After suitable incubation, the DCA solution has been discharged and the cells exposed to UVA irradiation and soon after read in the fluorimeter.

The cell viability after UVA exposure and without UV exposure is checked by NRU (Neutral red uptake) assay, a method able to discriminate alive, damaged or dead cells. Cells are incubated with scalar concentrations of the halogenated oligopeptide and with the Neutral Red solution (NR). After incubation, the medium is replaced with fresh medium + NR medium and cells are incubated at 37°C for 4 h. Then cells are washed several times to eliminate exceeding dye wastes and read at the colorimeter.

Results are shown in figure 14 A, B. No cytotoxic effect are observed at the tested concentrations, while a reduction of ROS release, indicative of an antioxidant effect, is observed in a dose dependent manner.

SEQUENCE LISTING

<110> Politecnico di Milano

<120> Elastomeric peptide

<130> E0117229

<160> 8

<170> PatentIn version 3.5

<210> 1
<211> 15
<212> PRT
<213> Artificial Sequence

<220>
<223> recombinant protein from Drosophila melanogaster gene CG15920

<400> SEQ ID no. 1

```
Gly Gly Arg Pro Ser Asp Ser Tyr Gly Ala Pro Gly Gly Gly Asn
1               5                   10                  15
```

<210> 2
<211> 11
<212> PRT
<213> Artificial Sequence

<220>
<223> fragment from Anopheles gambiae resilin

<400> SEQ ID no. 2

```
Ala Gln Thr Pro Ser Ser Gln Tyr Gly Ala Pro
1               5                   10
```

<210> 3
<211> 15
<212> PRT
<213> Artificial Sequence

<220>
<223> fragment Drosophila melanogaster resilin

<400> SEQ ID no. 3

Gly Gly Arg Pro Ser Asp Ser Tyr Gly Ala Pro Gly Gly Gly Asn
1 5 10 15

<210> 4
<211> 15
<212> PRT
<213> Artificial Sequence

<220>
<223> resilin consensus sequence

<400> SEQ ID no. 4

Ser Asp Thr Tyr Gly Ala Pro Gly Gly Gly Asn Gly Gly Arg Pro
1 5 10 15

<210> 5
<211> 7
<212> PRT
<213> Artificial Sequence

<220>
<223> repeat unit recombinant resilin

<220>
<221> misc_feature
<222> (2)..(3)
<223> Xaa can be any charged or polar amino acid

<220>
<221> misc_feature
<222> (6)..(6)
<223> Xaa can be any charged or polar amino acid

<400> SEQ ID no. 5

Ser Xaa Xaa Tyr Gly Xaa Pro
1 5

<210> 6
<211> 7
<212> PRT
<213> Artificial Sequence

<220>
<223> Resilin exon I consensus sequence

<400> SEQ ID no. 6

Ser Asp Ser Tyr Gly Ala Pro
1 5

<210> 7
<211> 8
<212> PRT
<213> Artificial Sequence

<220>

<223> SJXXZGXP

<220>
<221> misc_feature
<222> (2)..(2)
<223> Jaa can be a serin or it is absent

<220>
<221> misc_feature
<222> (3)..(4)
<223> Xaa can be any naturally occurring amino acid

<220>
<221> misc_feature
<222> (5)..(5)
<223> Zaa can be a tyrosine, histidine, phenylalanine or tryptophan

<220>
<221> misc_feature
<222> (7)..(7)
<223> Xaa can be any naturally occurring amino acid

<400> SEQ ID no. 7

```
Ser Jaa Xaa Xaa Zaa Gly Xaa Pro
1               5
```

<210> 8
<211> 7
<212> PRT
<213> Artificial Sequence

<220>
<223> SDSY*GAP

<400> SEQ ID no. 8

```
Ser Asp Ser Tyr Gly Ala Pro
1               5
```

## Claims

1. An oligopeptide of sequence SDSY*GAP (SEQ ID no. 8) wherein D is aspartic acid, Y* is a modified tyrosine, A is alanine wherein said modified tyrosine is mono or di-halogenated, and wherein the at least one halogen atom is selected from Cl, Br, I, preferably wherein the modified tyrosine is di-brominated.

2. The oligopeptide according to claim 1, wherein said tyrosine is di-*ortho*-brominated.

3. A biocompatible fiber having rigid and elastomeric properties comprising an oligopeptide according to claim 1 or 2.

4. An oligopeptide according to claim 1 or 2 for use in regenerative medicine.

5. A cosmetic formulation comprising an oligopeptide according to claim 1 or 2.

6. A thermoplastic elastomer composition comprising an oligopeptide according to claim 1 or 2.

## Patentansprüche

1. **1.** Oligopeptid der Sequenz SDSY*GAP (SEQ ID NO. 8), wobei D Asparaginsäure ist, Y* ein modifiziertes Tyrosin ist, A Alanin ist, wobei das modifizierte Tyrosin mono- oder dihalogeniert ist, und wobei das mindestens eine Halogenatom ausgewählt ist aus Cl, Br, I, bevorzugt wobei das modifizierte Tyrosin dibromiert ist.

2. **2.** Oligopeptid nach Anspruch 1, wobei das Tyrosin di-*ortho*-bromiert ist.

3. **3.** Biokompatible Faser mit starren und elastomeren Eigenschaften, umfassend ein Oligopeptid nach Anspruch 1 oder 2.

4. **4.** Oligopeptid nach Anspruch 1 oder 2 zur Verwendung in regenerativer Medizin.

5. **5.** Kosmetische Formulierung, umfassend ein Oligopeptid nach Anspruch 1 oder 2.

6. **6.** Thermoplastische Elastomer-Zusammensetzung, umfassend ein Oligopeptid nach Anspruch 1 oder 2.

## Revendications

1. **1.** Oligopeptide ayant la séquence SDSY*GAP (SEQ ID no. 8) où D est l'acide aspartique, Y* est une tyrosine modifiée, A est l'alanine, dans lequel ladite tyrosine modifiée est mono- ou di-halogénée, et dans lequel ledit au moins un atome d'halogène est choisi parmi Cl, Br, I, de préférence, dans lequel la tyrosine modifiée est dibromée.

2. **2.** Oligopeptide selon la revendication 1, dans lequel ladite tyrosine est di-*ortho*-bromée.

3. **3.** Fibre biocompatible ayant des propriétés rigides et élastomères, comprenant un oligopeptide selon la revendication 1 ou 2.

4. **4.** Oligopeptide selon la revendication 1 ou 2, destiné à être utilisé en médecine régénératrice.

5. **5.** Formulation cosmétique comprenant un oligopeptide selon la revendication 1 ou 2.

6. **6.** Composition élastomère thermoplastique comprenant un oligopeptide selon la revendication 1 ou 2.

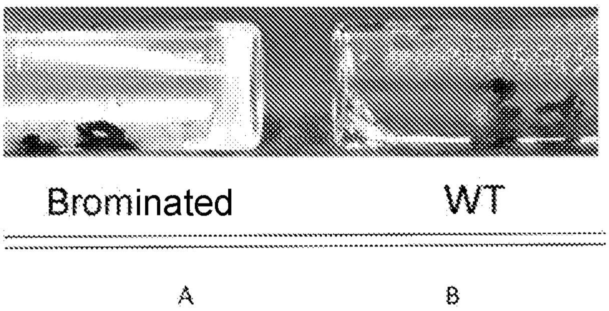


FIG. 1

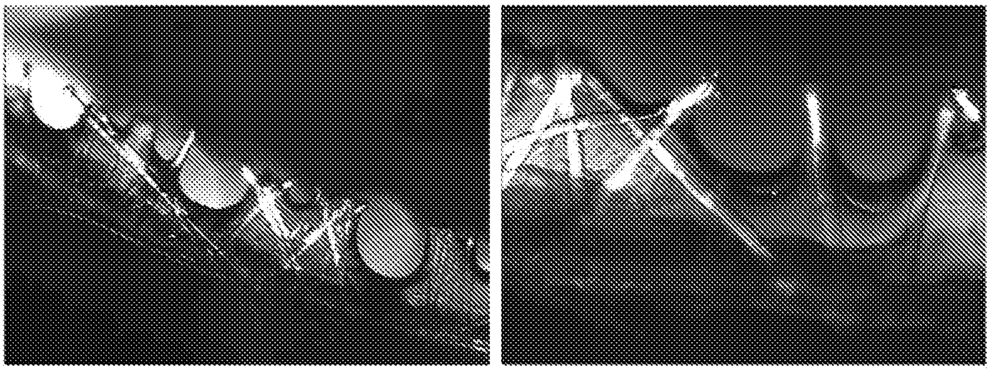

FIG. 2A

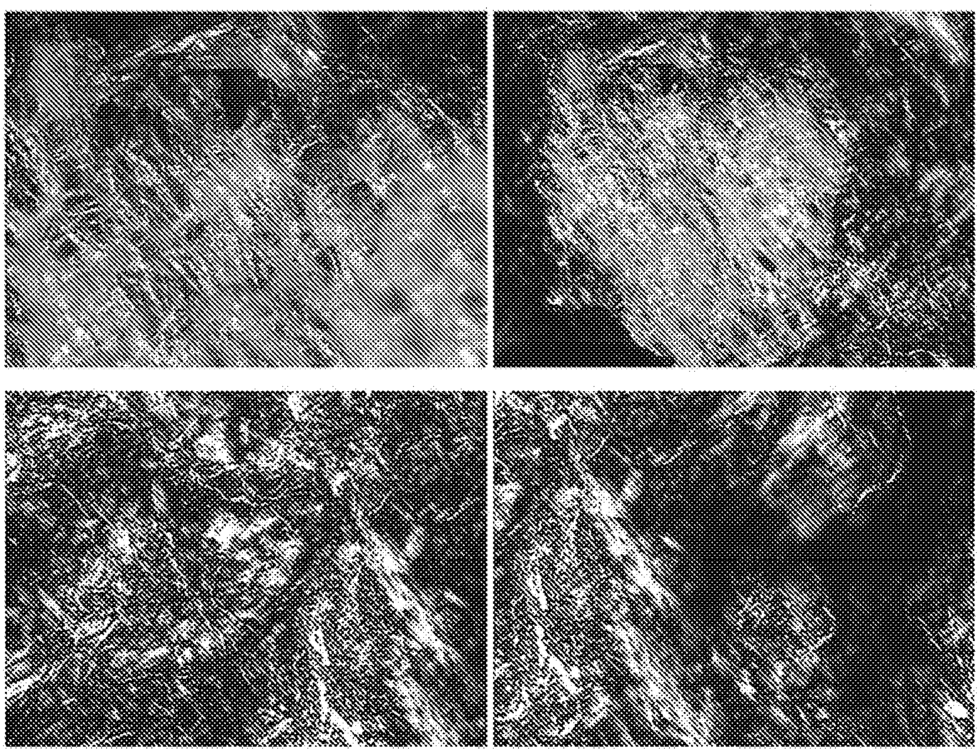

FIG. 2B

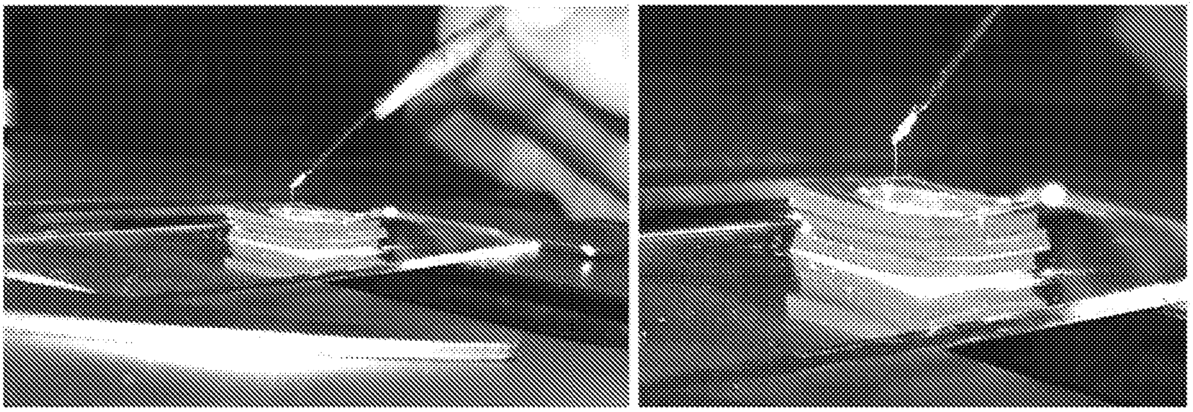

FIG. 3

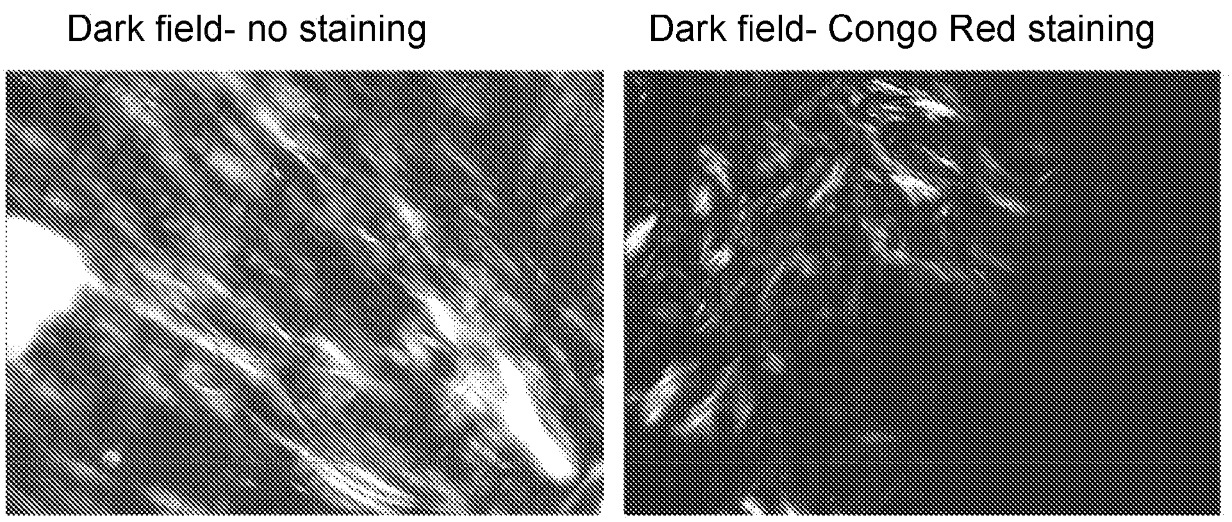


FIG. 4

FIG. 5

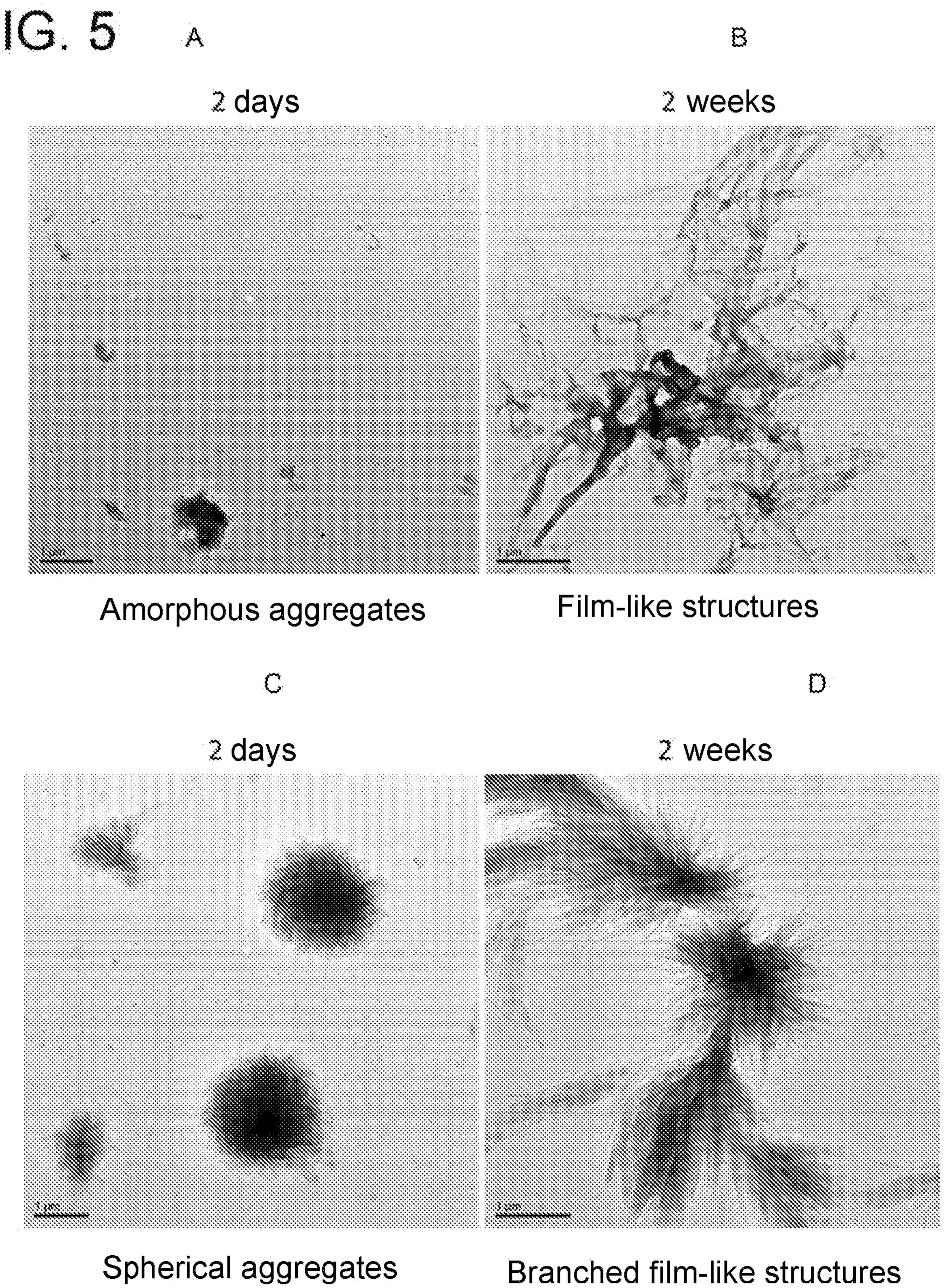
A
B
2 days
2 weeks
Amorphous aggregates
Film-like structures
C
D
2 days
2 weeks
Spherical aggregates
Branched film-like structures

FIG. 5

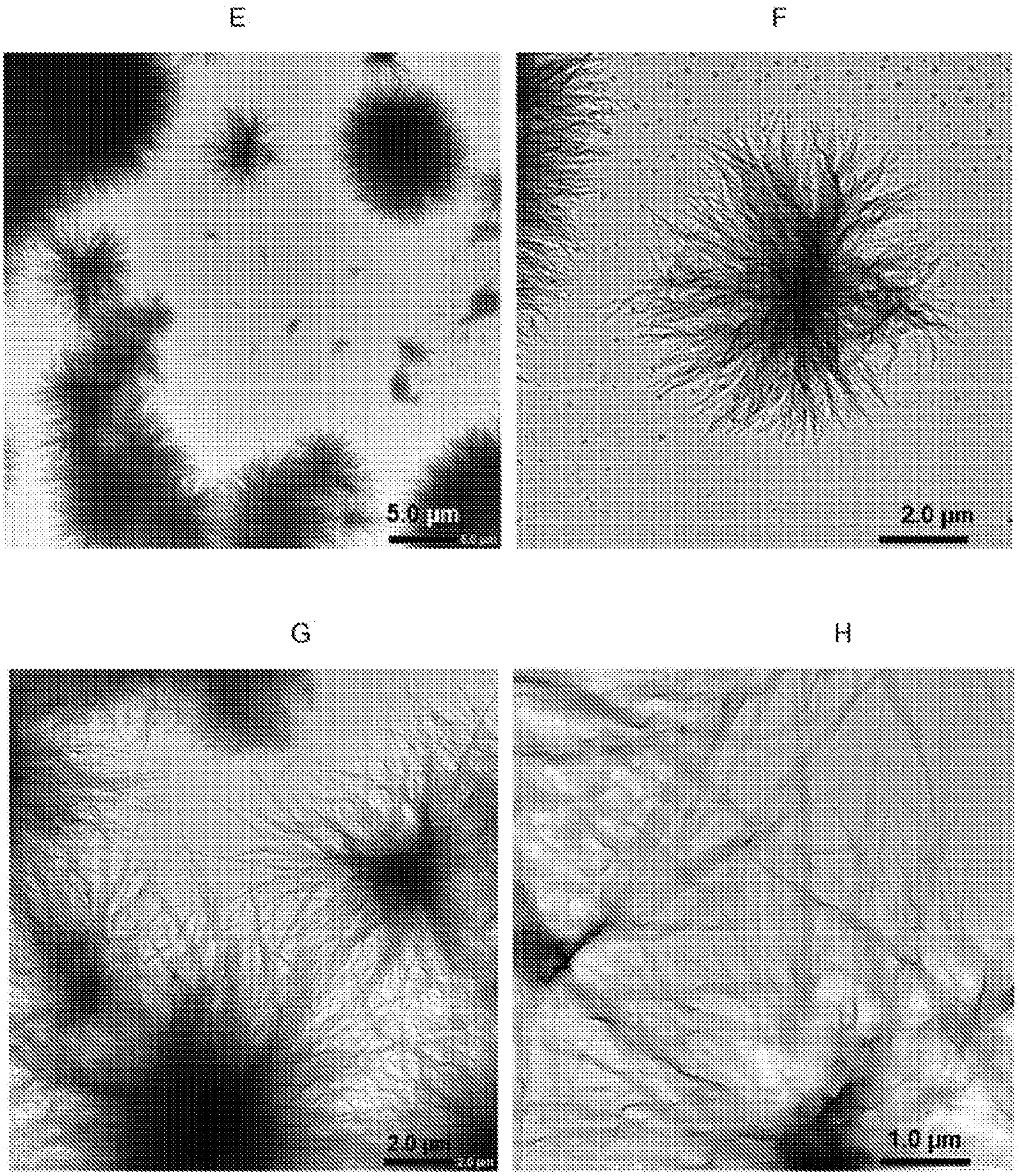

FIG. 5

I L

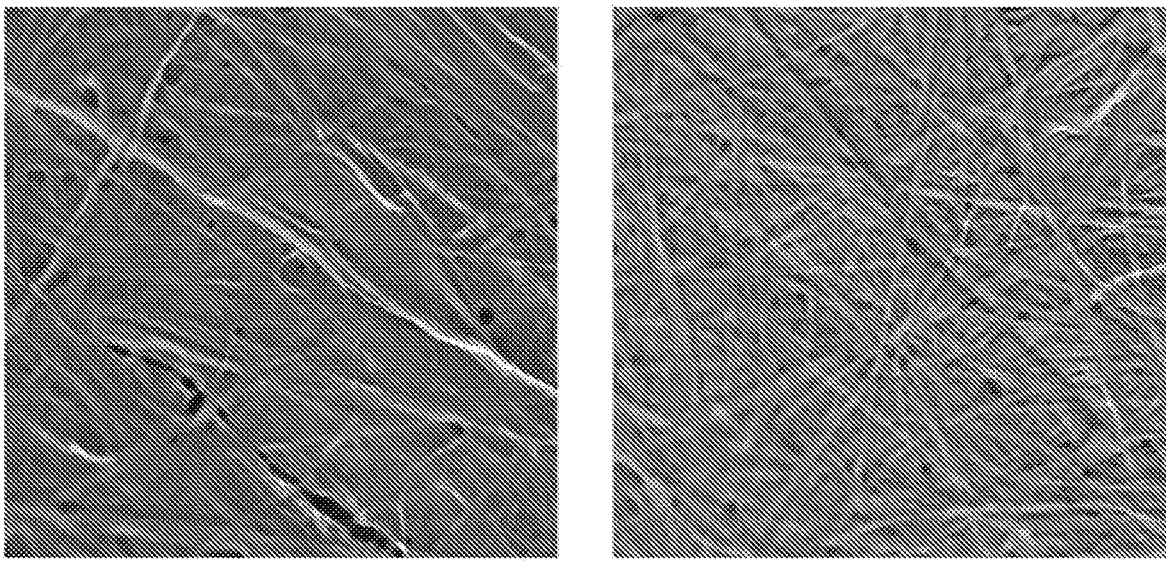

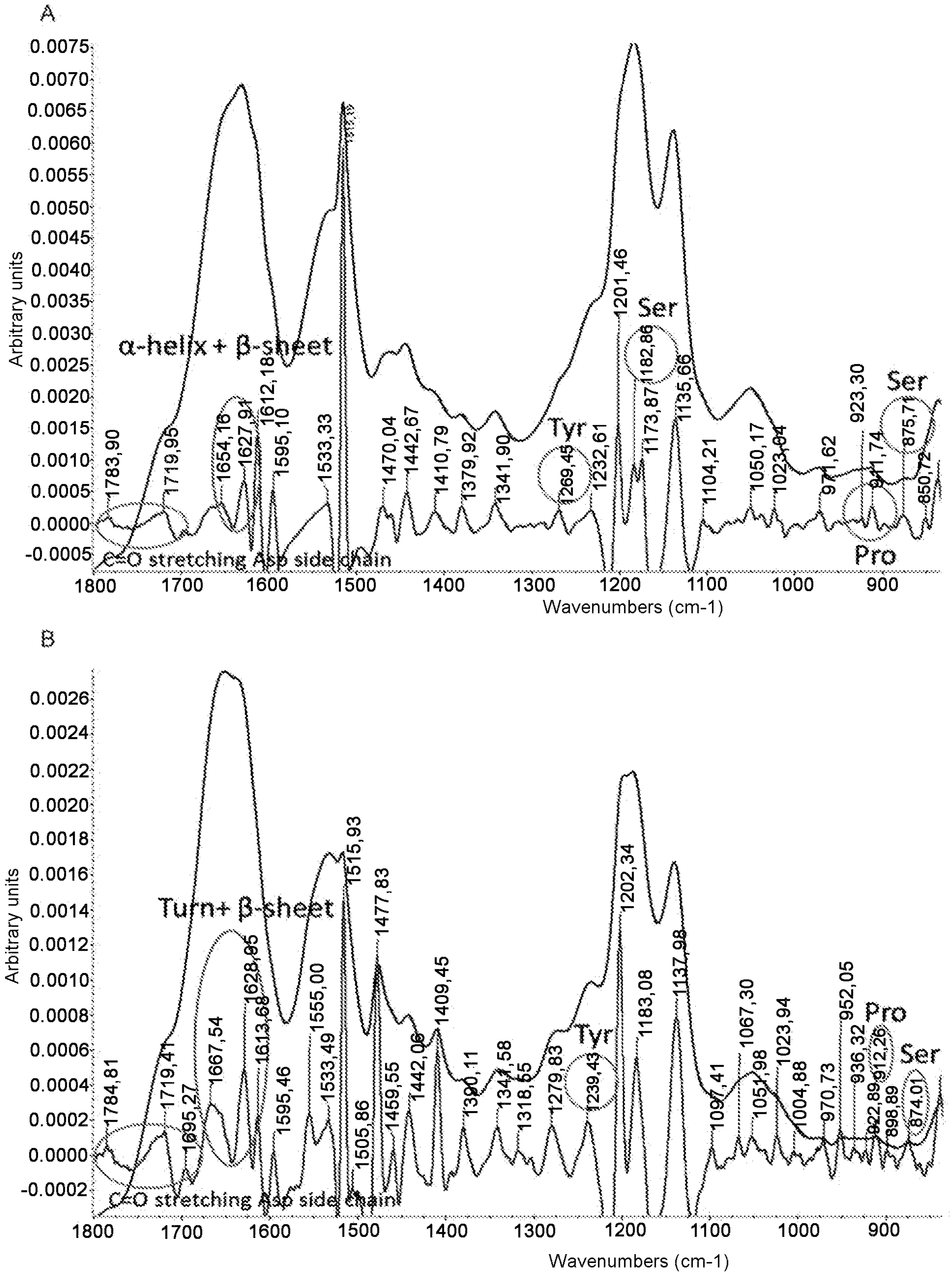
A
Arbitrary units
α-helix + β-sheet
Ser
Tyr
Pro
C=O stretching Asp side chain
Wavenumbers (cm-1)
B
Arbitrary units
Turn+ β-sheet
Tyr
Pro
Ser
C=O stretching Asp side chain
Wavenumbers (cm-1)

FIG. 6

A

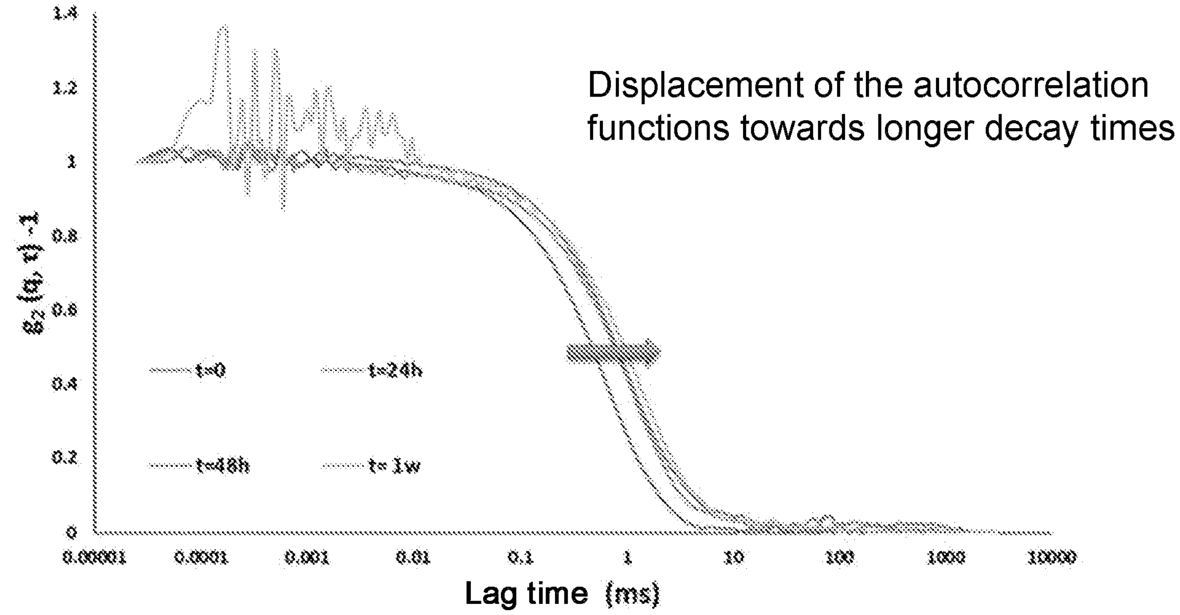
Displacement of the autocorrelation functions towards longer decay times
1.4
1.2
1
0.8
0.6
0.4
0.2
0
$g_2$ (q, τ) -1
t=0
t=24h
t=48h
t= 1w
0.00001
0.0001
0.001
0.01
0.1
1
10
100
1000
10000
Lag time (ms)

B

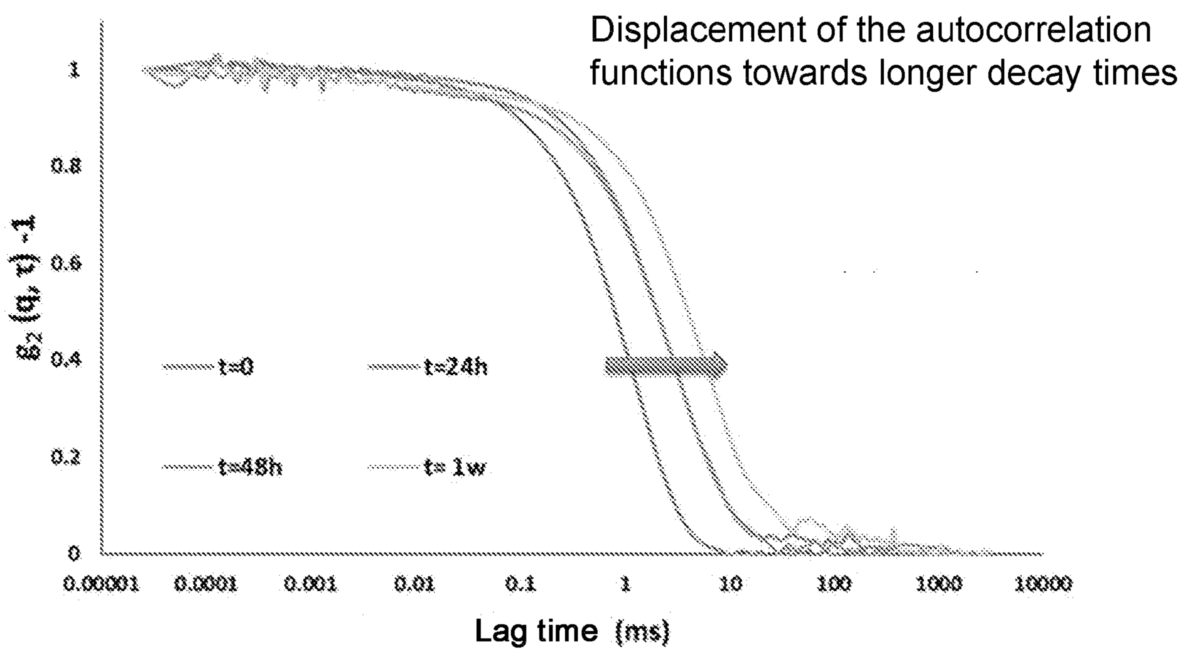
Displacement of the autocorrelation functions towards longer decay times
1
0.8
0.6
0.4
0.2
0
$g_2$ (q, τ) -1
t=0
t=24h
t=48h
t= 1w
0.00001
0.0001
0.001
0.01
0.1
1
10
100
1000
10000
Lag time (ms)

FIG. 7

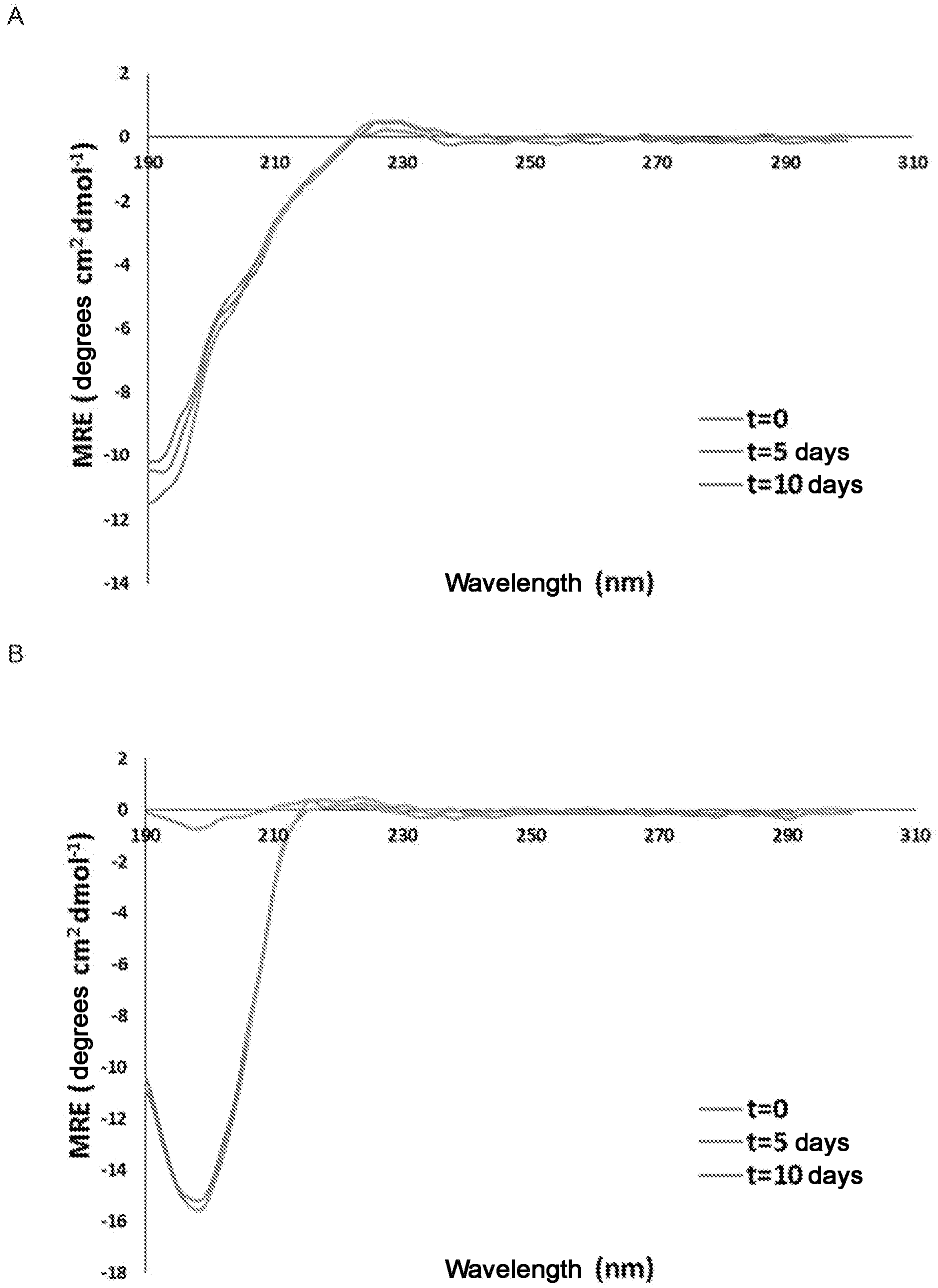
A
2
0
-2
-4
-6
-8
-10
-12
-14
190
210
230
250
270
290
310
MRE ( degrees cm$^2$ dmol$^{-1}$)
t=0
t=5 days
t=10 days
Wavelength (nm)
B
2
0
-2
-4
-6
-8
-10
-12
-14
-16
-18
190
210
230
250
270
290
310
MRE ( degrees cm$^2$ dmol$^{-1}$)
t=0
t=5 days
t=10 days
Wavelength (nm)

FIG. 8

A

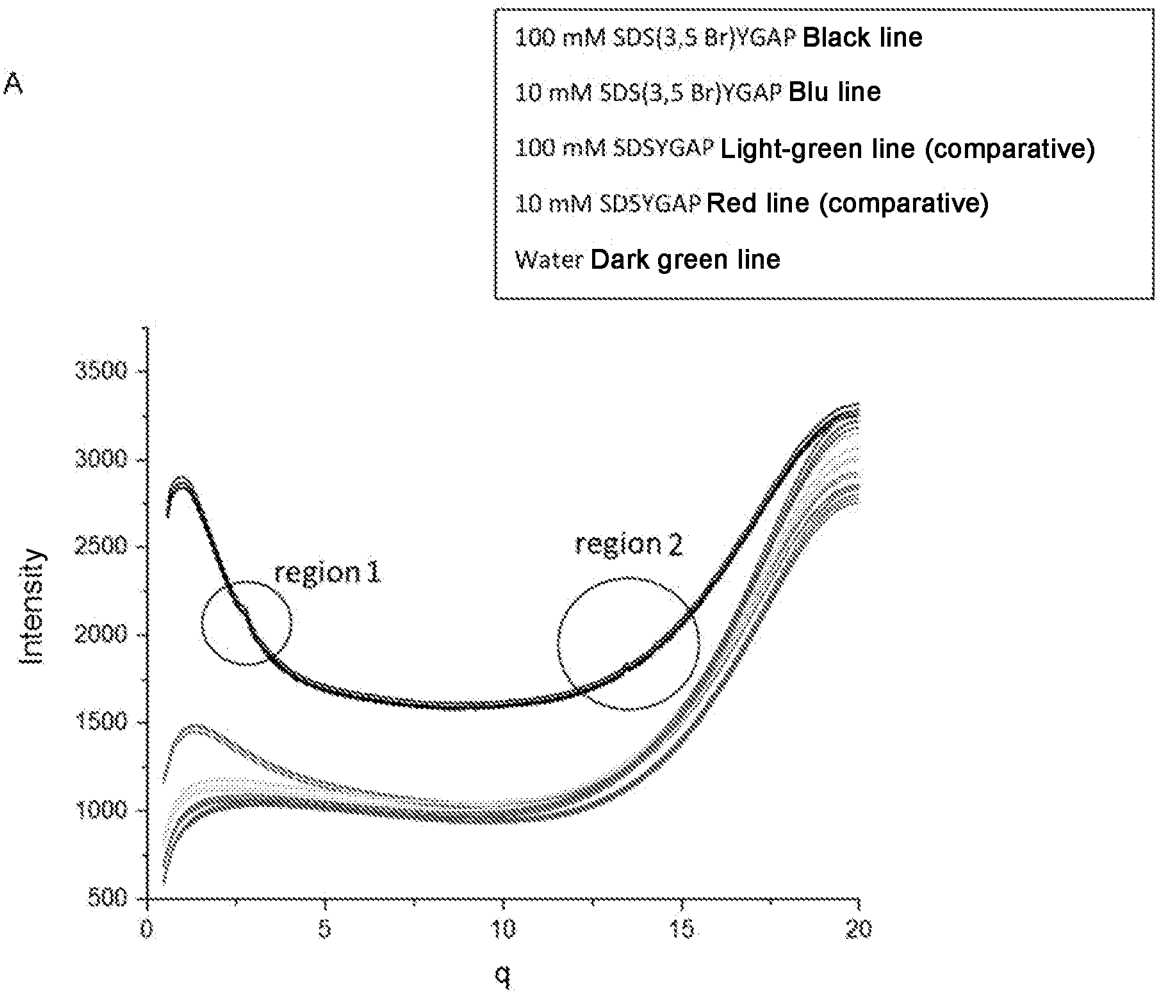
100 mM SDS(3,5 Br)YGAP Black line
10 mM SDS(3,5 Br)YGAP Blu line
100 mM SDSYGAP Light-green line (comparative)
10 mM SDSYGAP Red line (comparative)
Water Dark green line
Intensity
3500
3000
2500
2000
1500
1000
500
0
5
10
15
20
q
region 1
region 2

B

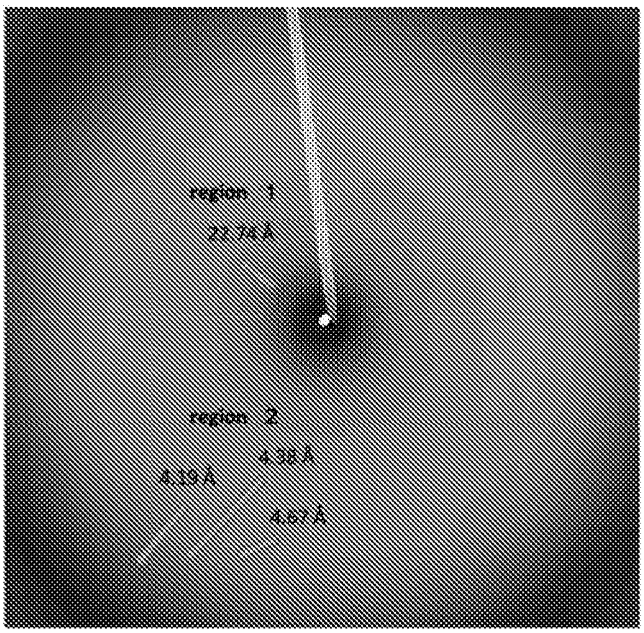
region 1
22.74 Å
region 2
4.38 Å
4.19 Å
4.67 Å

FIG. 9

FIG. 10

A

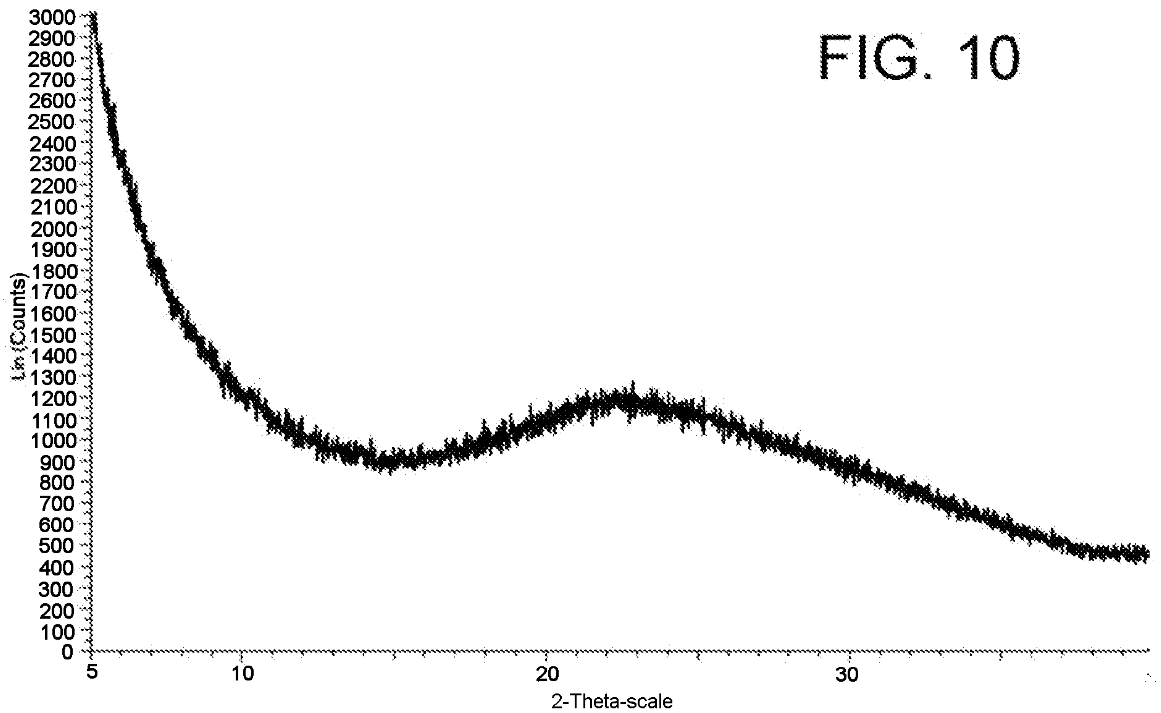


Background-File;background raw-Type:2Th/Th locked-Start:5000*-End:40.000*-Step:0.015*-Step time:102s-Temp:27°C Time started:9s-2-Theta:5000*-Theta:2.500*-Chi:0.00-Phi:0.00*-x:0.0mn

– Background: Silicon crystals

B

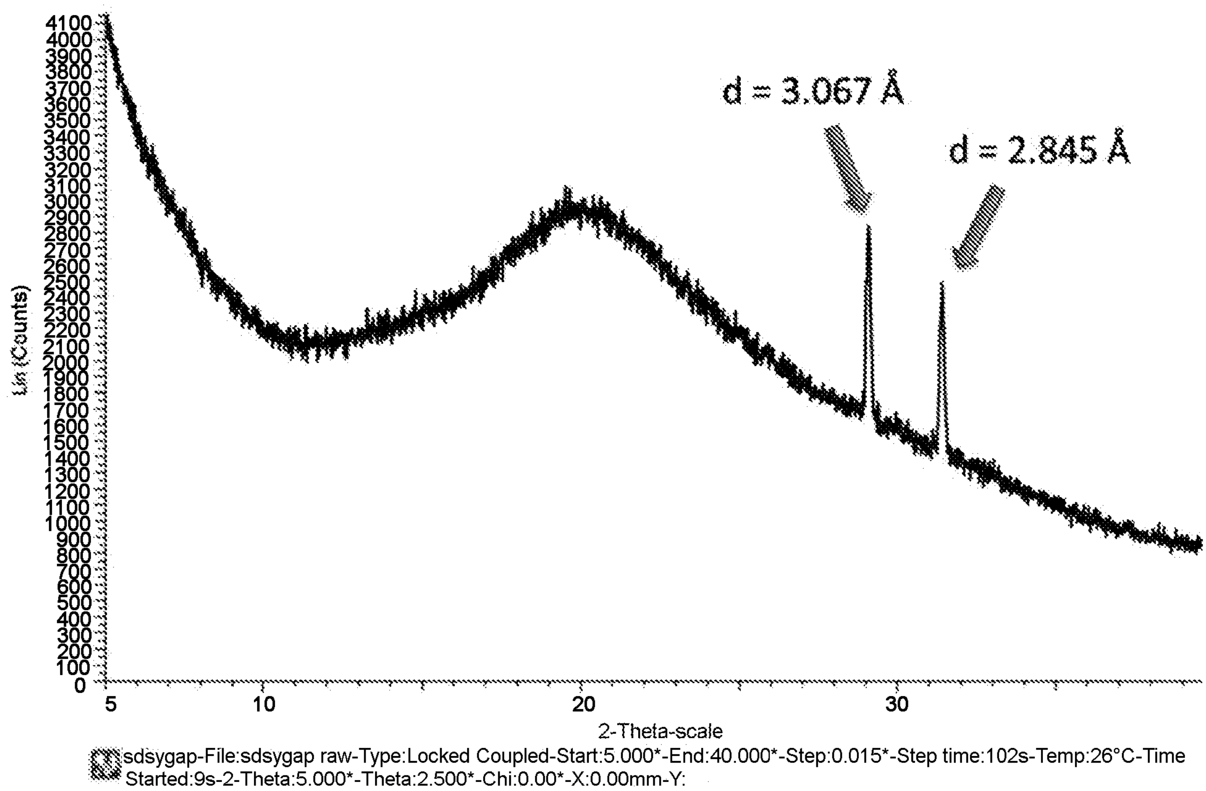


sdsygap-File:sdsygap raw-Type:Locked Coupled-Start:5.000*-End:40.000*-Step:0.015*-Step time:102s-Temp:26°C-Time Started:9s-2-Theta:5.000*-Theta:2.500*-Chi:0.00*-X:0.00mm-Y:

– SDSYGAP (1) –

C

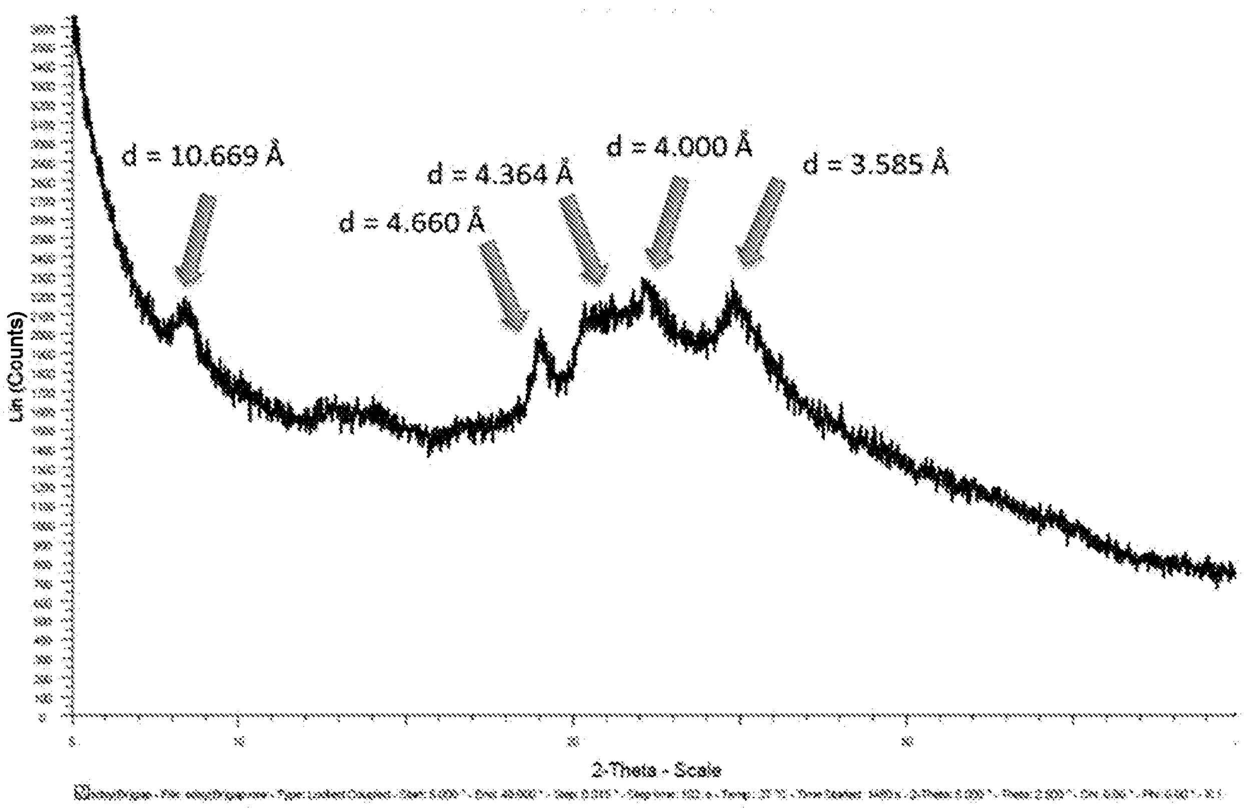


– SDSY(3,5-Br)GAP (**2**) –

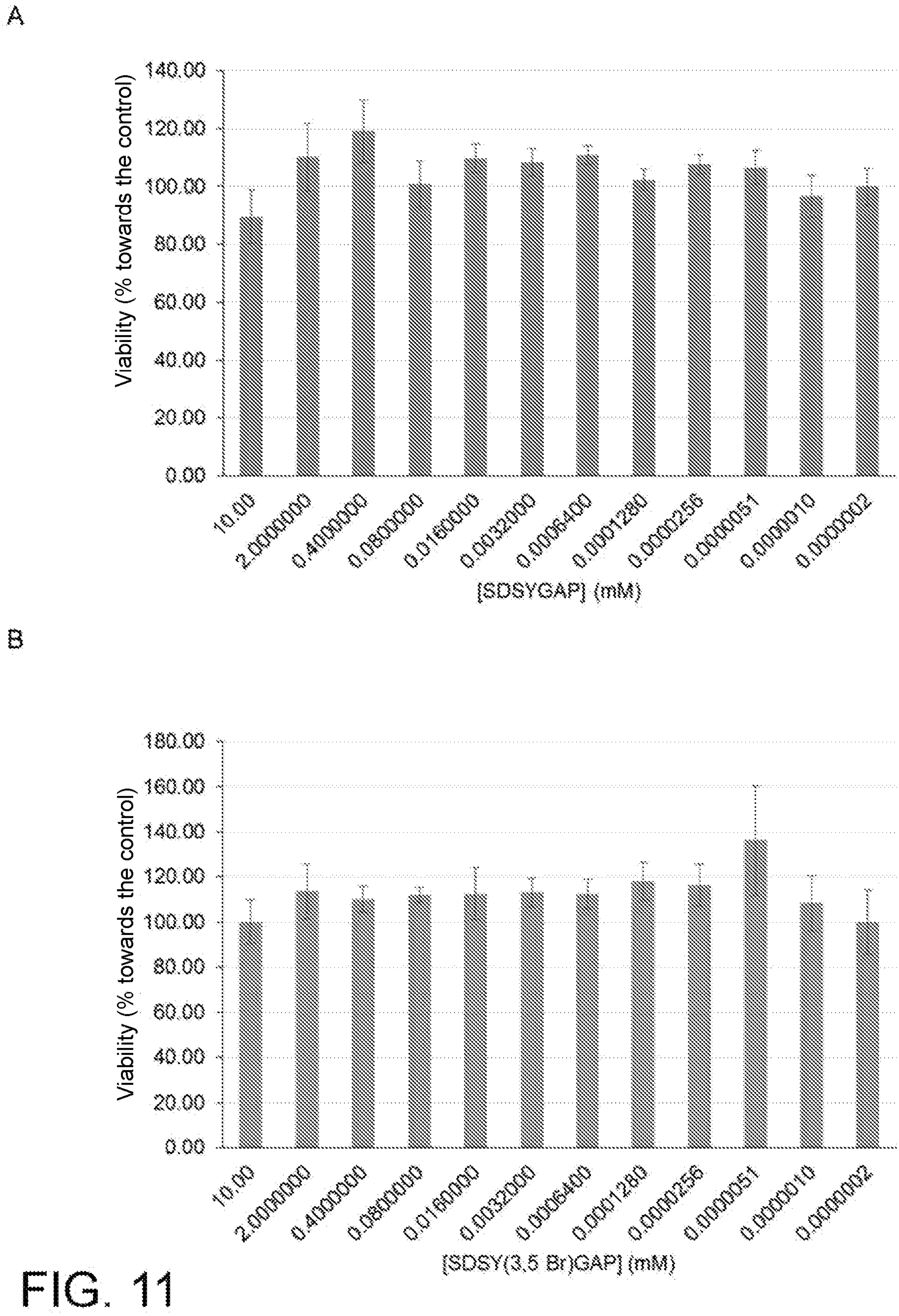
A
Viability (% towards the control)
140.00
120.00
100.00
80.00
60.00
40.00
20.00
0.00
10.00
2.0000000
0.4000000
0.0800000
0.0160000
0.0032000
0.0006400
0.0001280
0.0000256
0.0000051
0.0000010
0.0000002
[SDSYGAP] (mM)
B
Viability (% towards the control)
180.00
160.00
140.00
120.00
100.00
80.00
60.00
40.00
20.00
0.00
10.00
2.0000000
0.4000000
0.0800000
0.0160000
0.0032000
0.0006400
0.0001280
0.0000256
0.0000051
0.0000010
0.0000002
[SDSY(3,5 Br)GAP] (mM)

FIG. 11

FIG. 12

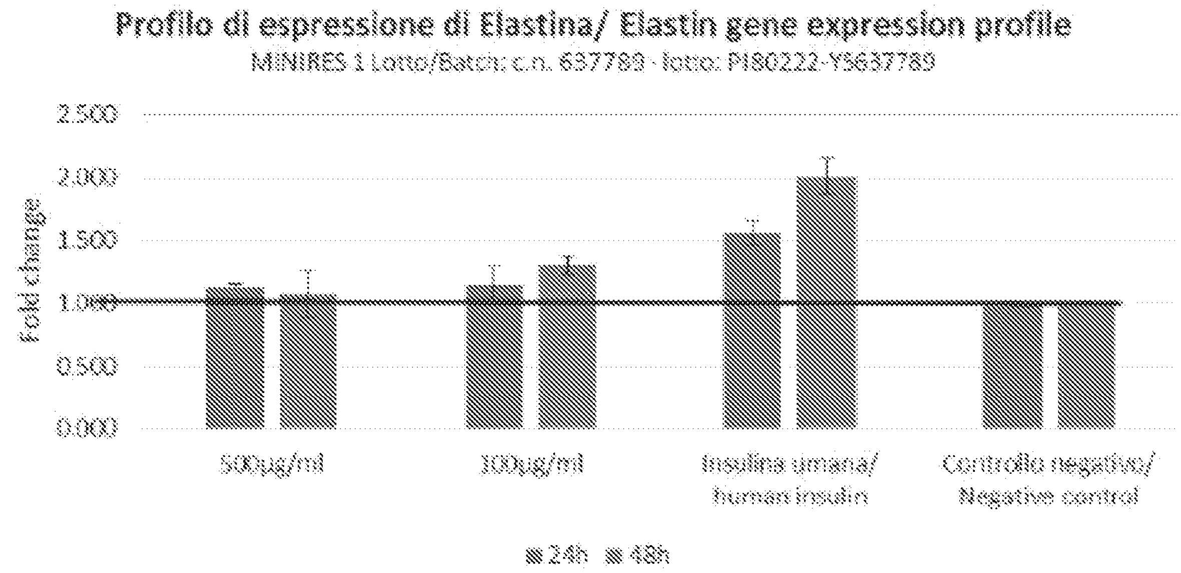


A

FIG. 13

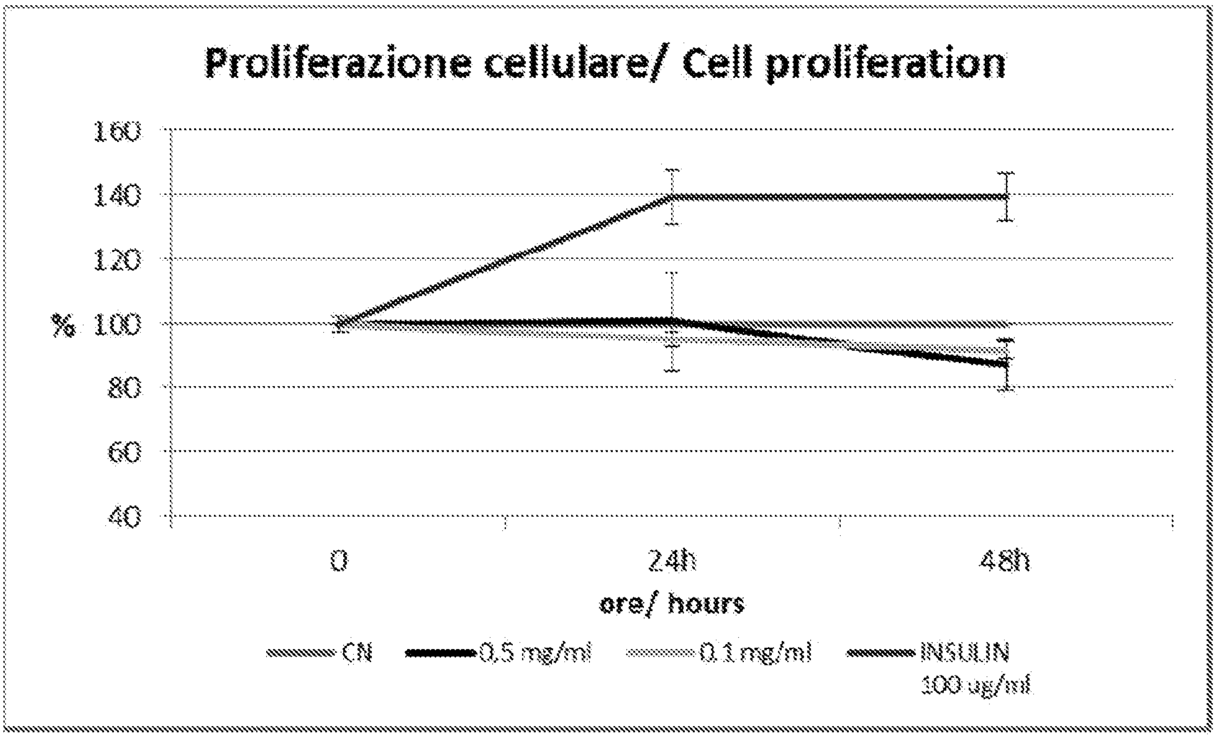

B

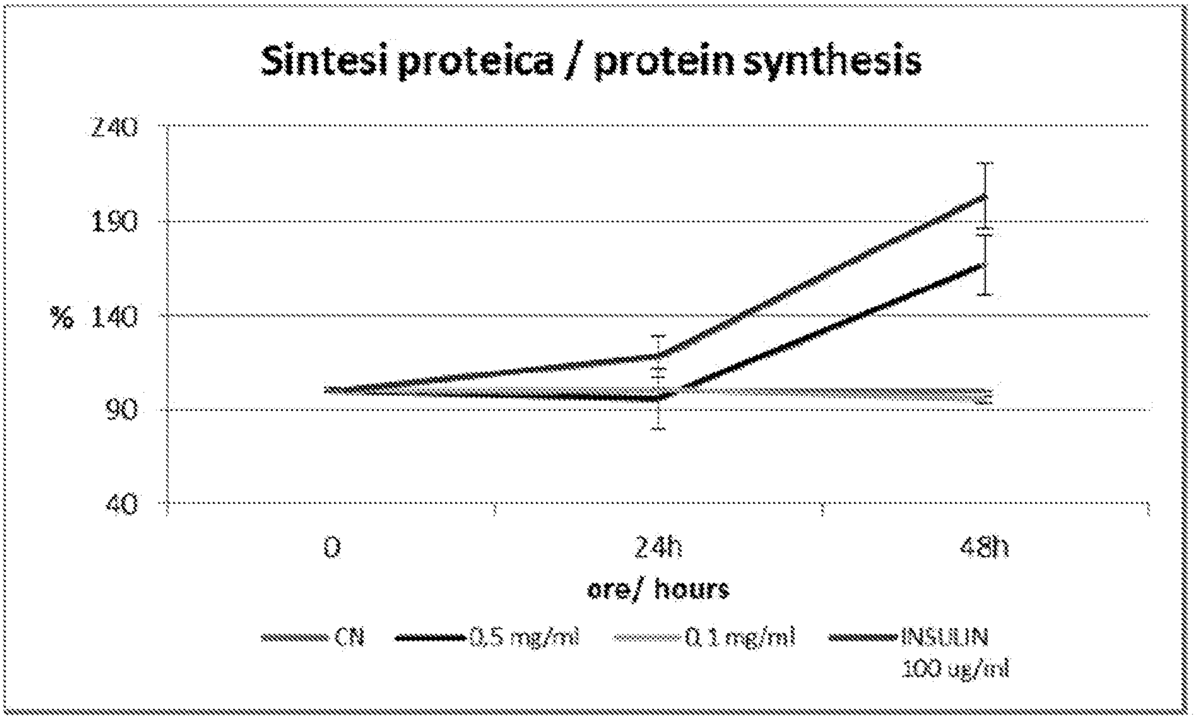
Sintesi proteica / protein synthesis
240
190
%
140
90
40
0
24h
48h
ore/ hours
CN
0,5 mg/ml
0,1 mg/ml
INSULIN 100 ug/ml

FIG. 14

A

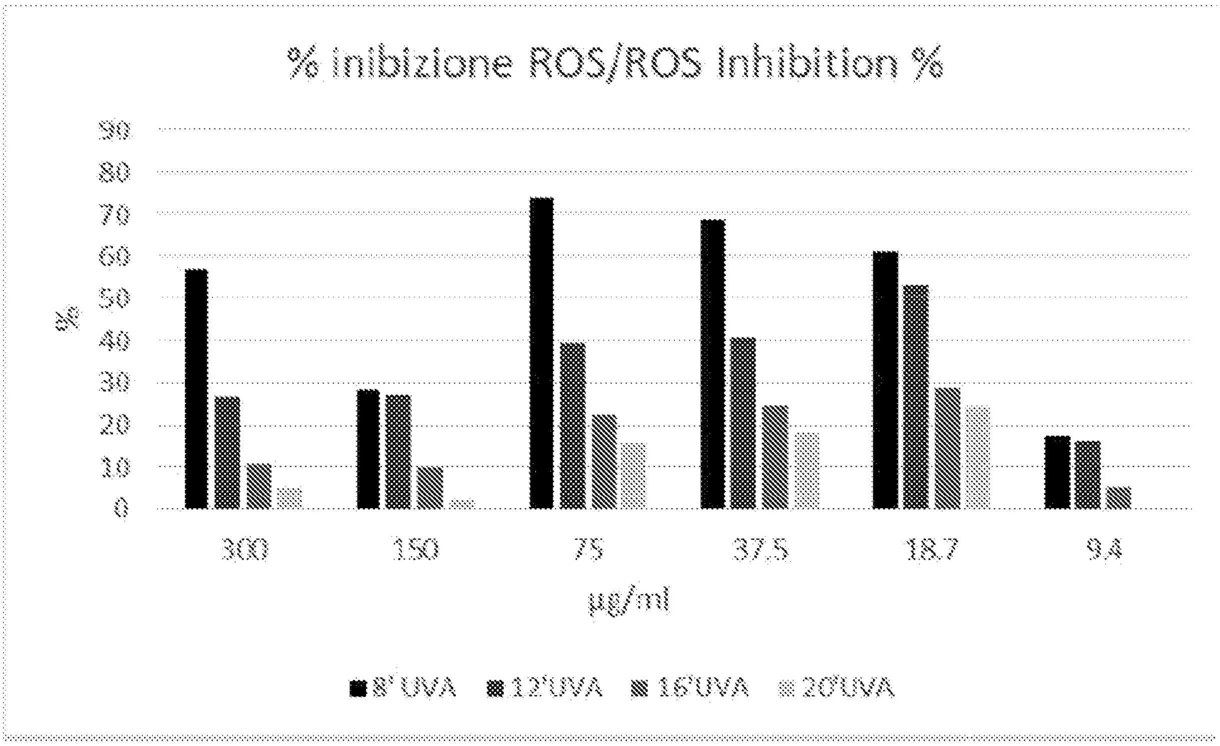
% inibizione ROS/ROS Inhibition %
90
80
70
60
50
40
30
20
10
0
%
300
150
75
37,5
18,7
9,4
µg/ml
8' UVA
12'UVA
16'UVA
20'UVA

B

| | Esposizione Exposure | MINIRES 1 Lotto/Batch: c.n. 637789 - lotto: PI80222-YS637789 µg/ml | | | | | | vit C 150 µg/ml |
|---|---|---|---|---|---|---|---|---|
| % Vitalità cellulare/% cell viability | | **300** | **150** | **75** | **37,5** | **18,7** | **9,4** | |
| | **20' UVA** | 99 | 99 | 99 | 97 | 96 | 91 | 97 |
| | **NO UVA** | 100 | 99 | 101 | 101 | 99 | 99 | 104 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004104042 A
- US 20100015070 A
- US 20160279295 A
- WO 2004104043 A
- WO 2009069123 A


### Non-patent literature cited in the description


- **ELVIN CM et al.** Synthesis and properties of crosslinked recombinant pro-resilin. *Nature,* 2005, vol. 437, 999-1002
- **TRUONG MY et al.** A pH-responsive interface derived from resilin-mimetic protein Rec1-resilin. *Biomaterials,* 2010, vol. 31, 4434-4446
- **CHARATI MB et al.** Hydrophilic elastomeric biomaterials based on resilin-like polypeptides. *Soft Matter,* 2009, vol. 5, 3412-3416
- **TAMBURRO AM et al.** Localizing α-Helices in Human Tropoelastin: Assembly of the Elastin "Puzzle. *Biomacromolecules,* 2009, vol. 10, 3009-3014
- **RENNER JN et al.** Characterization of Resilin-Based Materials for Tissue Engineering Applications. *Biomacromolecules,* 2012, vol. 13, 3678-3685
- **FLAMIA R et al.** AFM Study of the Elastin-like Biopolymer Poly(ValGlyGlyValGly). *Biomacromolecules,* 2011, vol. 12, 2957-2965
- **SBRANA F et al.** Multiscale characterization of a chimeric biomimetic polypeptide for stem cell culture. *Bioinspir. Biomim.,* 2012, vol. 7, 046007
- **SHIN H et al.** Biomimetic materials for tissue engineering. *Biomaterials,* 2003, vol. 24, 4353-5364
- **BERTOLANI A et al.** Supramolecular amplification of amyloid self-assembly by iodination. *Nature Communications,* 2015, vol. 6, 7574